(19) Europäisches Patentamt
European Patent Office
Office européen des brevets

(11) EP 1 720 994 B1

(12) FASCICULE DE BREVET EUROPEEN

(45) Date de publication et mention de la délivrance du brevet:
**15.04.2009 Bulletin 2009/16**

(21) Numéro de dépôt: **05730715.9**

(22) Date de dépôt: **23.02.2005**

(51) Int Cl.:
*C12P 19/04* (2006.01) *C08B 37/10* (2006.01)
*A61K 31/727* (2006.01) *A61K 31/702* (2006.01)
*A61P 35/00* (2006.01) *A61P 35/04* (2006.01)

(86) Numéro de dépôt international:
**PCT/FR2005/000431**

(87) Numéro de publication internationale:
**WO 2005/090591 (29.09.2005 Gazette 2005/39)**

# (54) OLIGOSACCHARIDES, PROCEDE DE PREPARATION, UTILISATION ET COMPOSITIONS PHARMACEUTIQUES LES RENFERMANT

OLIGOSACCHARIDE, HERSTELLUNGSVERFAHREN UND VERWENDUNG DAFÜR SOWIE SIE ENTHALTENDE PHARMAZEUTISCHE ZUSAMMENSETZUNGEN

OLIGOSACCHARIDES, PREPARATION METHOD AND USE THEREOF, AND PHARMACEUTICAL COMPOSITIONS CONTAINING SAME

(84) Etats contractants désignés:
**AT BE BG CH CY CZ DE DK EE ES FI FR GB GR HU IE IS IT LI LT LU MC NL PL PT RO SE SI SK TR**
Etats d'extension désignés:
**AL BA HR LV MK YU**

(30) Priorité: **24.02.2004 FR 0401810**

(43) Date de publication de la demande:
**15.11.2006 Bulletin 2006/46**

(73) Titulaire: **Aventis Pharma S.A.**
**92160 Antony (FR)**

(72) Inventeurs:
- **VISKOV, Christian**
  **F-91130 Ris Orangis (FR)**
- **MOURIER, Pierre**
  **F-94220 Charenton le Pont (FR)**

(74) Mandataire: **Morel-Pécheux, Muriel et al**
**sanofi-aventis**
**Département Brevets**
**174 avenue de France**
**75013 Paris (FR)**

(56) Documents cités:
**EP-A- 0 394 971**
**WO-A-81/01004**
**WO-A-90/01501**
**WO-A-94/14851**
**US-A- 4 847 338**
**US-A- 4 916 219**
**US-A- 5 106 734**

- **M. PETITOUT ET AL.: "Is there a unique sequence in heparin for interaction with heparin cofactor II?" THE JOURNAL OF BIOLOGICAL CHEMISTRY, vol. 263, no. 18, 1988, pages 8685-8690, XP008051888 cité dans la demande**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 1 720 994 B1

## Description

La présente invention concerne de nouveaux composés chimiques, particulièrement de nouveaux oligosaccharides, leur procédé de préparation, leur utilisation et des compositions pharmaceutiques les renfermant. Ces oligosaccharides sont utiles pour le traitement du cancer, en particulier la prévention et l'inhibition de la formation de métastases.

Plus particulièrement, l'invention concerne, selon un premier aspect, un procédé de dépolymérisation d'un polysaccharide ayant à l'origine des propriétés anti-thrombotiques.

Des procédés de dépolymérisation de polysaccharides à propriétés anti-thrombotiques sont connus. Ces procédés ont en commun :

- de viser à obtenir des oligosaccharides de masse moléculaire moyenne inférieure, afin de limiter les effets secondaires qui surviennent lorsque les polysaccharides de départ sont utilisés en tant que médicaments ;
- de maintenir une activité antithrombotique satisfaisante après dépolymérisation.

Les polysaccharides à propriétés anti-thrombotiques commerciaux tels que l'héparine ou les héparines de bas poids moléculaire telles que l'Enoxaparine, la tinzaparine, la fragmine, sont tous des inhibiteurs de l'héparanase.

Dans les conditions physiologiques normales, les cellules expriment l'enzyme héparanase. Cette enzyme permet de réguler indirectement la mitogènèse, la néo-vascularisation et la réparation des tissus. Un des mécanismes d'action de l'heparanase est de cliver le protéoglycane de sulfate d'héparane (l'heparan sulfate protéoglycan (HSPG)). Ce glycosaminoglycane est présent à la surface des cellules endothéliales et assure la cohésion de la membrane basale (matrice extracellulaire). Le clivage du protéoglycane de sulfate d'héparane entraîne la libération de facteurs de croissance tel que le FGF2. Le relargage des facteurs de croissance est nécessaire pour la mitogénèse et l'angiogénèse. Cependant, ceci n'est pas suffisant pour déclencher ces mécanismes biologiques, il est nécessaire que le FGF2 se fixe à un glycosaminoglycane pour générer une modification allostérique de la protéine et favoriser son interaction avec son récepteur. En fait, par le clivage de l'HSPG, l'héparanase génère des fragments de sulfate d'héparane qui vont se fixer au FGF2 et favoriser l'interaction avec ses récepteurs et ainsi induire les mécanismes biologiques mentionnés ci-dessus. Le clivage de l'HSPG de la matrice extracellulaire et la déstructuration des capillaires permet l'extravasation cellulaire.

L'héparanase est surexprimée par les cellules tumorales et favorise donc les métastases et leur néovascularisation. Ces phénomènes sont essentiels pour la propagation et la survie de la tumeur cancéreuse.

L'héparine, un glycosaminoglycane structurellement proche du sulfate d'heparane est connu pour être un inhibiteur puissant de l'héparanase. Cet effet a été longtemps attribué à la présence de la séquence spécifique de liaison à l'ATIII. En effet, cette séquence, bien que moins présente dans le sulfate d'héparane, est commune à ces deux glycosaminoglycanes. La zone de clivage de l'héparanase est représentée ci-après :

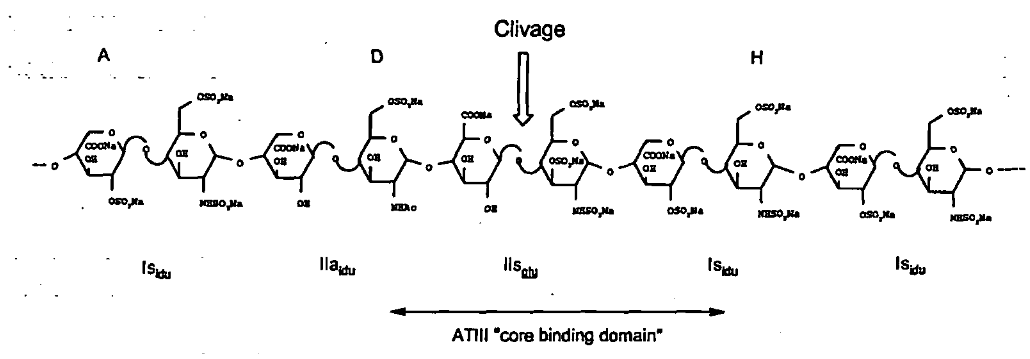


Le point de clivage de cette β-endo glycosidase est situé au centre de la séquence minimale de fixation à l'ATIII. On peut donc penser que les propriétés antithrombotiques et d'inhibition de l'héparanase sont intimement liées (en fait l'héparine est un substrat compétitif du sulfate d'héparane). De ce fait, il est difficile d'utiliser ce glycosaminoglycane comme anti-métastatique. En effet, ses fortes propriétés anti-coagulantes limitent sa marge thérapeutique et induisent des effets secondaires graves tels que des hémorragies sévères. De plus, l'injection répétée d'héparine peut entraîner dans certains cas des thrombocytopénies entraînant une issue fatale (réaction immunologique lié à l'association entre l'héparine et le facteur plaquettaire 4 (PF4)).

Peu de documents mettent en lumière les liens entre la structure des oligosaccharides et leurs propriétés anti-héparanase, en corrélation avec une activité anti-métastatique. Ainsi, Bitan et al. (Isr. J. Med. Sci. 1995 ; 31 : 106-118) précise les conditions structurales requises pour l'inhibition de la colonisation de mélanomes pulmonaires par des

espèces d'héparine inhibant l'héparanase : L'héparanase est inhibée efficacement par des fragments d'héparine contenant 16 sucres ou plus (résumé ; fig.2, p.110; fig.3, p.111; p. 116, col. de droite, 2ème phrase). Les hexasaccharides sont décrits comme de mauvais inhibiteurs de l'héparanase (Fig.8, p.115). De plus, il est dit que l'inhibition de l'héparanase n'est possible qu'avec des molécules ayant une masse moléculaire supérieure ou égale à au moins 4000 daltons (résumé, p.116, col. de droite, 4éme phrase). Toutefois, la méthode de détermination de l'inhibition de l'héparanase est une méthode indirecte, puisqu'elle consiste en l'évaluation de l'aptitude de cellules à dégrader la matrice extra-cellulaire en présence des différents produits à tester (p.108 ; col. de droite, 2ème paragraphe « Degradation of Sulfated Proteoglycans »). Elle n'est donc pas spécifique de l'héparanase. De plus, tous les produis décrits sont obtenus par une méthode de clivage de l'héparine par voie chimique (acide nitreux) (p.108; col. de gauche, 2ème paragraphe « Heparin-Derived Oligosaccharides »).

Voir aussi :

Vlodavski I, et al. Modulation of neovascularization and metastasis by species of heparin, Heparin and related Polysaccharides, D.A. Lane, et al., Editor, Plenum Press, New York, 1992;

Parish C R, et al. Evidence that sulphated polysaccharides inhibit tumor metastasis by blocking tumor-cell-derived heparanases, Int. J. Cancer 40: 511-518, 1987.

A ce jour, il existe un besoin important en composés anti-métastatiques, et pour lequel il n'y a pas de solution commercialement acceptable. Les poly- et oligo-saccharides inhibiteurs d'héparanase connus actuellement sont issus directement de sources naturelles (héparine) ou de procédés plus ou moins difficiles à mettre en oeuvre (certaines héparines de bas poids moléculaire) et présentent une composante anti-thrombotique marquée, qui n'est pas compatible avec un traitement anticancéreux, notamment lorsque le patient à traiter est sensible aux hémorragies.

Un des problèmes actuels est donc d'obtenir un produit présentant une activité anti-héparanase significative, essentiellement dépourvu d'activité anti-thrombotique, via une méthode simple et reproductible.

A cette fin, et de façon surprenante, il a été trouvé qu'un nouveau procédé de dépolymérisation d'un polysaccharide ayant à l'origine des propriétés anti-thrombotiques, dans lequel le polysaccharide est dépolymérisé par l'héparinase I à une température comprise entre 10°C et 20°C jusqu'à ce que son activité anti-thrombotique, due notamment à l'inhibition des facteurs Xa et IIa, soit essentiellement éteinte (<35 UI/mg), permet d'obtenir un produit conservant une activité anti-héparanase significative.

Ce procédé constitue donc un moyen efficace d'obtention de produits enrichis en site anti-héparanase du polysaccharide, tout en éliminant sa composante anti-thrombotique. Le procédé est plus avantageusement exploité lorsque la dépolymérisation du polysaccharide est poursuivie jusqu'à ce que son activité anti-thrombotique soit inférieure à 20 UI/mg.

Plus particulièrement, la dépolymérisation est poursuivie jusqu'à atteindre une masse moléculaire moyenne inférieure à 5000 Da, de préférence inférieure à 3000 Da.

Contre toute attente, le produit obtenu par ce procédé simple à mettre en oeuvre, contient notamment des hexasaccharides qui sont de bons inhibiteurs de l'héparanase. De plus, ces hexasaccharides ont une masse moléculaire moyenne nettement inférieure à 4000 daltons, puisqu'elle est généralement comprise entre 1000 et 2000 daltons.

Le polysaccharide est de préférence une héparine.

La dépolymérisation est avantageusement poursuivie jusqu'à ce que le mélange de la fraction hexasaccharidique soit essentiellement exempt d'hexasaccharides sulfatés $\Delta$Is-Is$_{id}$-Is$_{id}$ et. $\Delta$Is-Is$_{id}$-IIS$_{glu}$.

Les enzymes sont habituellement utilisées dans des conditions dites « physiologiques », c'est à dire dans les conditions dans lesquelles elles fonctionnent habituellement *in-vivo* dans les organismes à partir desquels elles sont extraites (notamment: pH, température, force ionique, éventuellement cofacteurs physiques (lumière...) ou chimiques (coenzymes...). La plupart des enzymes peuvent être couramment utilisées à des températures supérieures à leur température physiologique, par exemple 45-50°C. Dans notre situation, et contre toute attente, il a été observé que la dépolymérisation peut encore avoir lieu à une température comprise entre 10 et 20°C, en particulier de 16°C, dans des conditions de sélectivité et de cinétique acceptables, préservant ainsi au mieux les composés inhibiteurs de l'héparanase formés au cours de la réaction de dépolymérisation. De plus, cela permet de limiter la concentration finale en héparinase I dans le milieu réactionnel en fin de réaction. En effet, la mise en oeuvre de la réaction à une température inférieure à la température optimale de réaction de l'héparinase I, qu'on peut situer vers 25-45°C, permet d'éviter de procéder à des ajouts excessifs d'enzyme en cours de réaction. Des ajouts d'enzyme sont habituellement effectués lorsqu'on observe une baisse de la cinétique de réaction autre que due à l'appauvrissement en substrat. Par conséquent, l'utilisation d'une température de réaction relativement basse permet indirectement de faciliter une éventuelle étape ultérieure de purification, notamment en raison de la présence limitée d'enzyme. La dépolymérisation peut donc de ce fait être conduite à une température comprise entre 10 et 20°C.

Afin d'éliminer d'éventuels oligosaccharides de faible masse moléculaire qui se seraient formés au cours de la dépolymérisation, en particulier des disaccharides et des tétrasaccharides, le procédé selon l'invention est poursuivi

avantageusement par une étape dans laquelle le produit de dépolymérisation du polysaccharide est purifié par chromatographie par perméation de gel (GPC) à un pH inférieur à 8 et supérieur à 5.

Le procédé selon l'invention comprend avantageusement une étape ultérieure de purification par chromatographie liquide haute performance (HPLC) dans laquelle une phase stationnaire, par exemple une silice, est une phase inverse greffée (i) en C18 et (ii) en cétyl triméthyl ammonium (CTA-SAX).

Le procédé comprend en outre une première étape de dessalage, comprenant avantageusement l'utilisation d'une phase mobile contenant un électrolyte en solution aqueuse, ledit électrolyte étant de préférence essentiellement transparent entre 200 et 250 nm. Des électrolytes acceptables comprennent NaCl, mais pour une utilisation avec un détecteur UV entre 200-250 nM, il est préférable d'utiliser perchlorates, méthanesulfonates ou phosphates de métaux alcalins tels que Na. Une phase stationnaire acceptable pour la première étape de dessalage est une résine échangeuse d'anions. Une résine particulièrement préférée est une résine Sepharose Q ®.

Le procédé peut en outre comprendre une seconde étape de dessalage, de préférence à l'aide d'un gel d'exclusion moléculaire, par exemple et de préférence de type Sephadex G10 ®.

Une autre solution pour détecter les produits selon l'invention dans les fractions collectées en sortie de colonne HPLC peut éventuellement consister en l'utilisation d'un diffractomètre.

D'autres techniques de dessalage acceptables incluent l'utilisation de techniques d'osmose, par exemple à l'aide de membranes polymériques.

Selon un second aspect, l'invention concerne des produits obtenus par un procédé conforme à son premier aspect.

Petitou et al. dans J. Biol. Chem. (1988), 263(18), 8685-8690 divulguent un hexasaccharide de formule $\Delta Is\text{-}II_{idu}\text{-}IIs_{glu}$ sous forme de se! de sodium isolé à partir d'un produit obtenu par un procédé de dépolymérisation partielle de l'héparine par l'héparinase I. Ce produit est décrit comme ne présentant pas d'activité anti-thrombotique. Aucune autre propriété de ce produit n'est mise en évidence. US 4 916 219 décrit aussi un procédé de dépolymérisation de l'héparine par l'héparinase. Ces deux procédés sont effectués à 30°C.

Selon un troisième aspect, l'invention concerne des produits de formule générale (I)

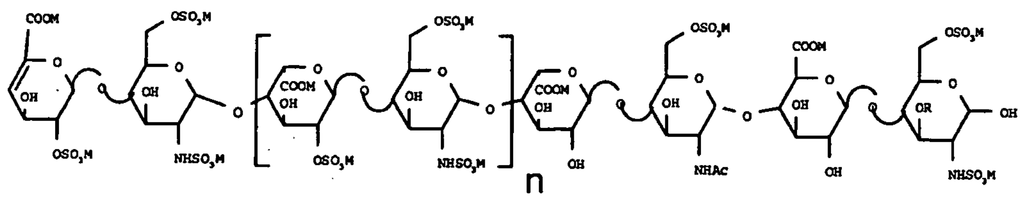

$\Delta Is$ $\quad Is_{idu}$ $\quad IIa_{idu}$ $\quad IIs_{glu}$

n = 0 à 5

dans laquelle :

R est choisi parmi H et $SO_3M$, et
M est choisi parmi H, Li, Na et K ;

à l'exception du produit pour lequel n = 0, R = $SO_3M$ et M = Na.

De façon inattendue, il a été observé que les produits conformes au troisième aspect de l'invention présentent de meilleures propriétés physico-chimiques lorsque M est choisi parmi Li, Na et K, de préférence Na. En particulier, la solubilité et la stabilité sont améliorées.

Des produits de formule générale (I) préférés sont ceux pour lesquels n = 0.

Selon un quatrième aspect, l'invention concerne des hexasaccharides. Un produit de formule (Ia) suivante :

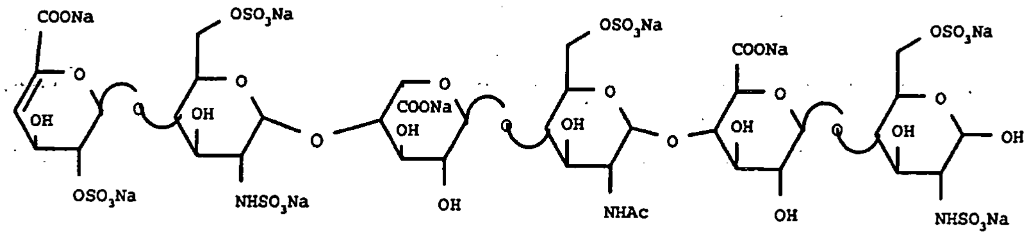

Δ Is IIa$_{idu}$ IIs$_{glu}$

est conforme à l'invention selon son quatrième aspect.

Un produit de formule (Ib) suivante :

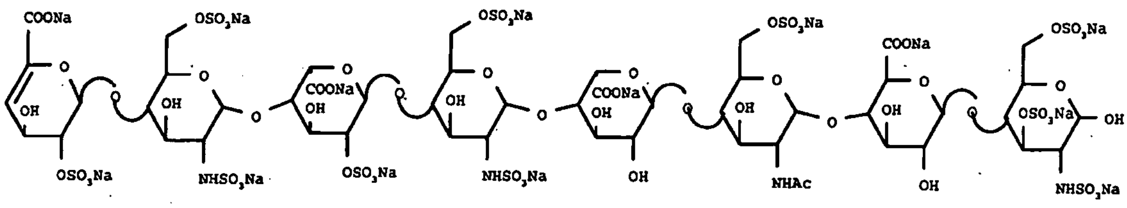

ΔIs Is IIa IIs

est conforme à l'invention selon son troisième aspect.

Un produit de formule (Ic) suivante :

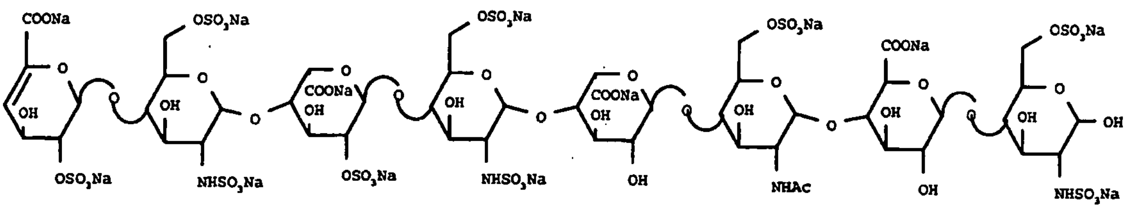

ΔIs Is IIa IIs

est conforme à l'invention selon son troisième aspect.

Un produit de formule (Id) suivante :

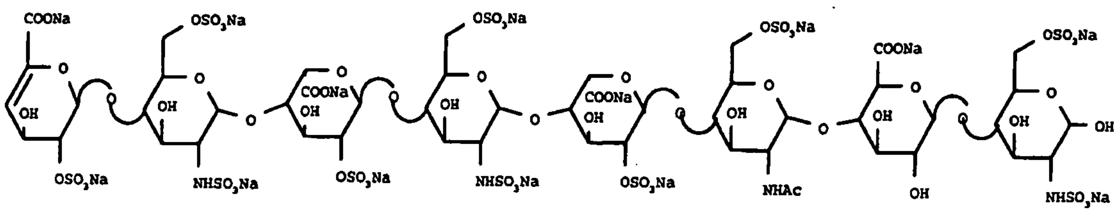

ΔIs Is Ia IIs

est conforme à l'invention.

Un produit de formule (Ie) suivante :

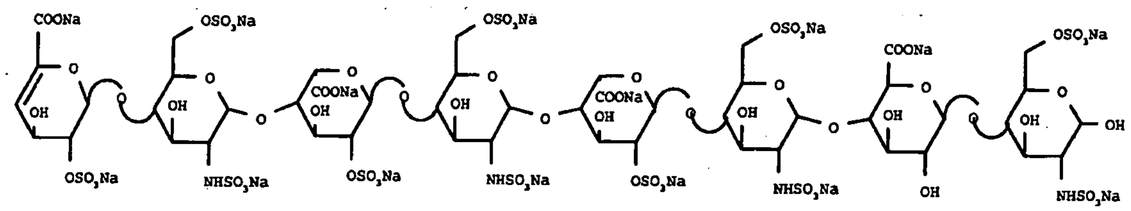

ΔIs Is Is IIs

est conforme à l'invention.

Un produit de formule (If) suivante:

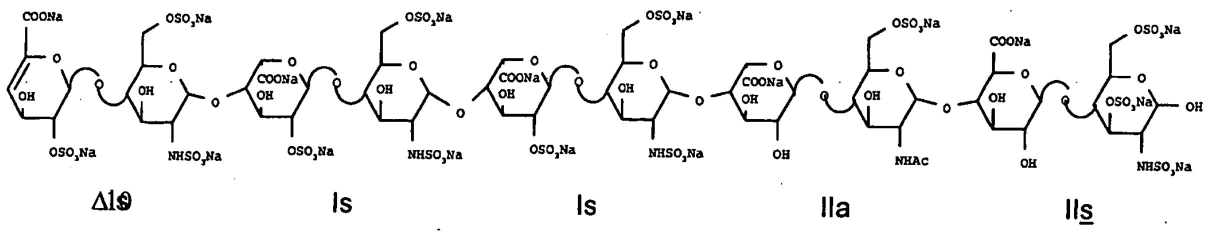

ΔIs Is Is IIa IIs

est conforme à l'invention selon son troisième aspect.

Un produit de formule (Ig) suivante :

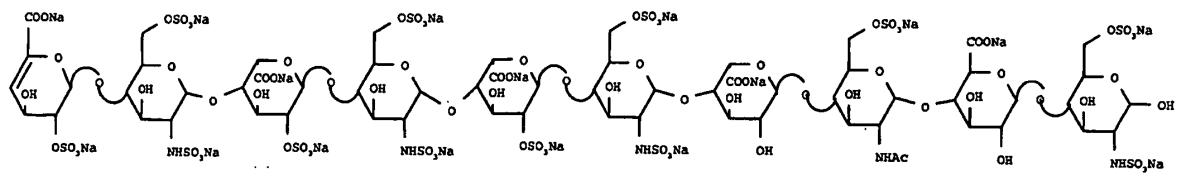

ΔIs Is Is IIa IIs

est conforme à l'invention selon son troisième aspect.

Un produit de formule (Ih) suivante :

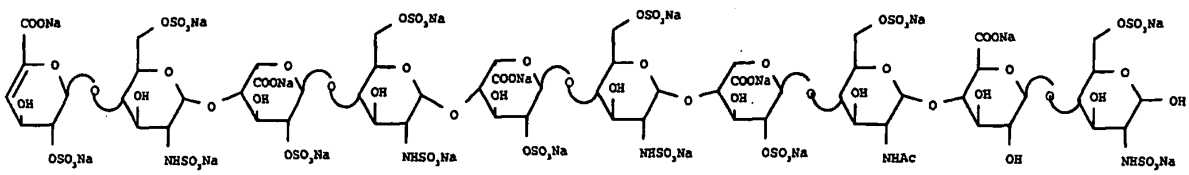

ΔIs Is Is Ia IIs

est conforme à l'invention.

Un produit de formule (Ij) suivante :

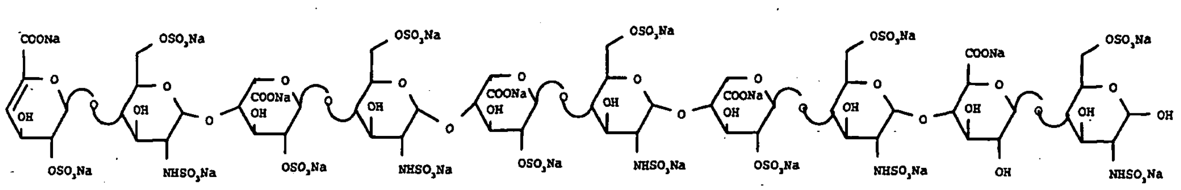

ΔIs Is Is Is IIs

est conforme à l'invention.

Un produit de formule (Ik) suivante :

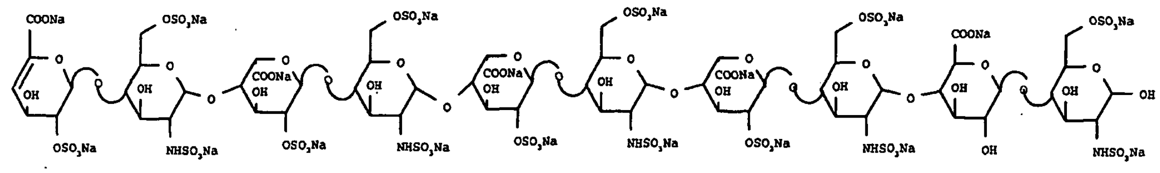

ΔIs Is Is Is IIs

est conforme à l'invention.

Un produit de formule (Im) suivante :

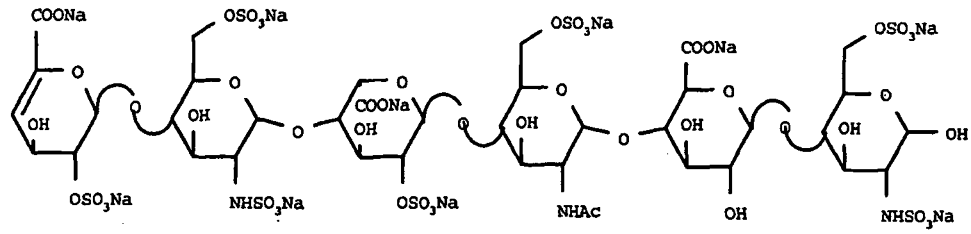

ΔIs Ia IIs

est conforme à l'invention selon son quatrième aspect.

Selon un cinquième aspect, l'invention concerne l'utilisation d'un produit selon l'un quelconque des second à quatrième aspects, pour moduler une prolifération cellulaire, en particulier liée au cancer, notamment un cancer du sein, du poumon, de la prostate, du colon ou du pancréas.

Une utilisation d'un produit selon le cinquième aspect de l'invention est particulièrement avantageuse lorsque que la prolifération cellulaire est liée à un processus métastatique, ainsi que lorsque l'utilisation est effectuée à un stade précoce de la maladie.

Une utilisation d'un produit selon le cinquième aspect de l'invention est particulièrement avantageuse en combinaison avec un second produit anticancéreux, de préférence cytotoxique.

Un second produit anticancéreux est avantageusement choisi parmi des dérivés du platine tels que le cisplatine ou l'oxaliplatine, des taxoïdes tels que le docétaxel ou le paclitaxel, des dérivés de bases puriques ou pyrimidiques telles que le 5-FU, la capécitabine ou la gemcitabine, des vincas tels que la vincristine ou la vinblastine, des moutardes, des hétérocycles aromatiques condensés tels que la staurosporine, l'éllipticine ou les camptothécines telles que l'irinotécan, le topotécan, des combrétastatines telles que la CA4P, des dérivés de la colchicine, tels que le phosphate de colchinol.

Le second produit anticancéreux est de préférence le docétaxel, l'oxaliplatine ou l'irinotécan.

Lorsque l'on étudie l'inhibition de l'heparanase par diverses héparines de bas poids moléculaire commerciales, on se rend compte qu'elles sont toutes inhibitrices de l'héparanase (Enoxaparine, tinzaparine, fragmine, etc ...). Cependant, nous avons pu constater qu'une nouvelle héparine de très bas poids moléculaire (ULMWH = Ultra Low Molecular Weight Heparin) (WO 02/08295; et la demande internationale non encore publiée PCT/FR03/02960) n'inhibe pas l'héparanase bien qu'elle comporte plus de séquences affines à l'ATIII que l'Enoxaparine. Il y a là par conséquent une

incohérence par rapport à la théorie énoncée plus haut.

Le procédé mis en oeuvre selon l'invention conduit notamment à la formation d'un hexasaccharide qui est l'IsoATIII, de structure Is-IIa-IIs, ci-dessous :

Hexasaccharide Is-IIa-IIs (Iso ATIII) :

A D

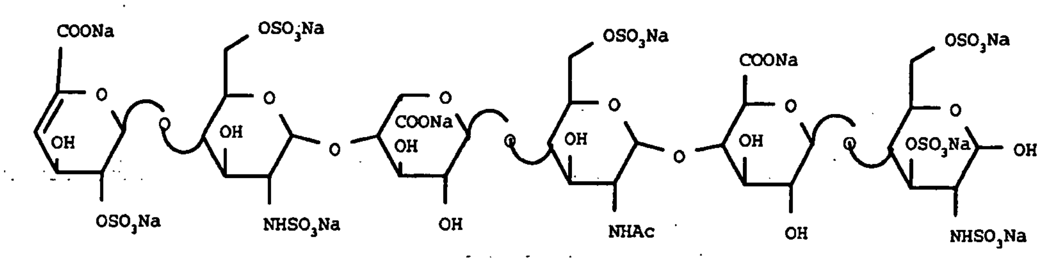

ΔIs IIa$_{idu}$ IIs$_{glu}$

Les résultats ci-après montrent que cet hexasaccharide Iso ATIII est un très bon inhibiteur d'héparanase. Il présente de plus l'avantage considérable de n'avoir aucune affinité à l'ATIII et par conséquent d'être dénué d'activité antithrombotique. L'avantage majeur de cette invention est la déconnection des propriétés antithrombotiques des héparinoides de leur propriétés inhibitrices de l'héparanase. Comparativement à l'héparine et aux HBPM, la marge thérapeutique de l'hexasaccharide Iso ATIII est fortement accrue et le rend potentiellement utisable comme agent antimétastatique. Dans la présente invention, nous revendiquons son utilisation en tant que tel et la préparation de l'hexasaccharide Iso ATIII seul ou en mélange avec d'autres hexasaccharides issus de la dépolymérisation controlée de l'héparine par l'héparinase I. De plus, nous revendiquons l'idée de dépolymériser l'héparine par l'heparinase I jusqu'au point d'extinction de son activité aXa et l'utilisation de ce mélange comme agent antimétastatique. Ce sera dans ce cas une HBPM non antithrombotique et specifiquement inhibitrice de l'héparanase.

Nous revendiquons la préparation et l'utilisation en tant qu'antimétastatique des produits suivants isolés ou en mélange :

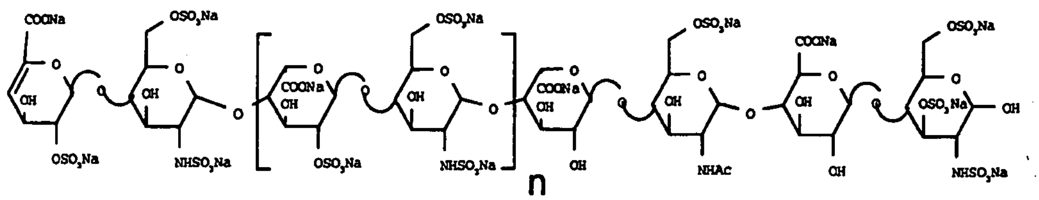

ΔIs Is$_{idu}$ IIa$_{idu}$ IIs$_{glu}$

n = 0 à 5

**Partie expérimentale**

**GPC**

La chromatographie d'exclusion sur gel est mise en oeuvre avec 2 colonnes de TSK Super SW2000 (300 x 4.6mm) et une colonne de garde de TSK Super (35 x 4.6 mm) (TOSOH BIOSEP). La détection est réalisée par absorptiométrie dans l'UV à 232 nm. La phase mobile est de l'acétate d'ammonium 0,1 M. Le volume injecté est de 5 μl.

**Chromatographie CTA-SAX**

Le suivi HPLC est réalisé par la méthode de CTA-SAX. La colonne utilisée est une Hypersil BDS (150 x 2,1 mm) particules 3 μm sur laquelle a été adsorbé du cétyl triméthyl ammonium par percolation d'une solution d'hydrogénophosphate de cétyl triméthyl ammonium 1mM dans un mélange eau - méthanol (68-32) v/v à 45°C à 0,2 ml/min pendant 4 heures.

Les conditions de séparation sur ce type de colonne sont les suivantes : la température de la colonne greffée est maintenue à 40°C. On procède à un gradient d'élution où le solvant A est de l'eau ajustée à pH 3 par ajout d'acide méthane sulfonique. Le solvant B est une solution 2N de méthane sulfonate d'ammonium ajusté à pH 2,6. Le gradient d'élution est le suivant :

| Temps (min) | Solvant A | Solvant B | Débit (ml/min) |
|---|---|---|---|
| 0 | 99 | 1 | 0.22 |
| 44 | 35 | 65 | 0.22 |
| 74 | 0 | 100 | 0.22 |

La détection utilisée est l'absorptiométrie dans l'UV à 232 nm. On utilise aussi 202-247 nm comme détection spécifique des oligosaccharides acétylés.

**Chromatographie semi-préparative sur CTA-SAX**

Chromatographie sur colonne Hypersil BDS (250 x 20 mm ) particules 5 µm, sur laquelle ont été greffées des chaînes cétyl triméthyl ammonium par percolation d'une solution d'hydrogénophosphate de cétyl triméthyl ammonium 1 mM dans un mélange eau-méthanol (68-32) v/v à 45°C à 2 ml/min pendant 4 heures.
La séparation est effectuée à température ambiante. Un gradient d'élution est utilisé : le solvant A est de l'eau mise à pH 2,5 par ajout de HCl. Le solvant B est une solution de NaCl 2N ajustée à pH 2,5.

| Temps (min) | Solvant A | Solvant B | Débit (ml/min) |
|---|---|---|---|
| 0 | 60 | 40 | 10 |
| 44 | 0 | 100 | 10 |

La détection est dans l'UV à 232 nm. On peut injecter 100 mg de fraction hexasaccharide à chaque séparation.

**Préparation de l'Hexasaccharide Iso ATIII**

L'hexasaccharide $\Delta$Is-IIa$_{id}$-$\underline{\text{IIs}}_{glu}$ (hexasaccharide iso ATIII) est obtenu par clivage par l'héparinase 1 du site affin ATIII de l'héparine. La dépolymérisation de l'héparine par l'héparinase 1 est endolytique : elle conduit à un mélange d'oligosaccharides insaturés sur leur extrémité non réductrice. A la fin de la réaction, on obtient un mélange de disaccharides, de tétrasaccharides et d'hexasaccharides. Toutes les zones les plus sulfatées de l'héparine sont clivées et transformées en disaccharides et en tétrasaccharides. Seules les parties acétylées restent sous forme d'hexasaccharides et surtout les enchaînements du type -GlcNS(6S ou 60H)-IdoA-GlcNAc(6S ou 6OH)-GlcA-GlcNS(3S ou 3OH, 6S ou 6OH)-

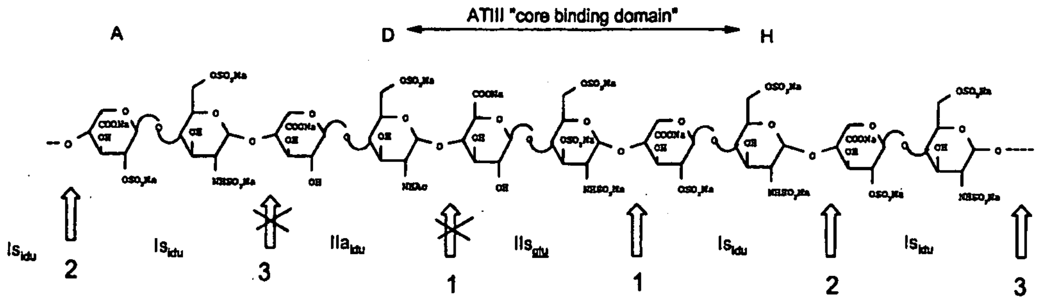


La dépolymérisation de l'héparine se fait dans les conditions suivantes : 3 g d'héparine de mucus de porc sont dissous dans 30ml d'une solution 0,2M NaCl, 0,02 % BSA, $Na_2HPO_4$ 5 mM ajustée à pH 7. La température de dépolymérisation est à 16°C. 2UI d'héparinase 1 sont initialement introduites. Au bout de 7 jours, une unité supplémentaire d'héparinase 1 est ajoutée. Au bout de 15 jours, la dépolymérisation de l'héparine est considérée achevée. Le suivi de la réaction est réalisé soit en GPC analytique sur colonne de TSK Super SW 2000 (Figure 1), soit sur colonne de CTA-SAX (Figure 2a). On peut considérer que la réaction enzymatique est suffisamment avancée lorsque la proportion d'oligosaccharides de taille supérieure à l'octasaccharide est limitée et lorsque les 2 principaux hexasaccharides sulfatés $\Delta$Is-$Is_{id}$-$Is_{id}$ et $\Delta$Is-$Is_{id}$-$IIs_{glu}$ du mélange ont été dépolymérisés en tétrasaccharides. Lorsque la réaction enzymatique est terminée, la solution est filtrée sur membrane 0,2 μm puis injectée en 2 fois sur une colonne de GPC remplie de Biogel P10 (Bio Rad) dans laquelle circule une phase mobile de NaCl 0,2N (Figure 3). L'hexasaccharide $\Delta$Is-$IIa_{id}$-$\underline{IIs}_{glu}$ est extrêmement fragile en milieu alcalin : il perd sa glucosamine terminale 3-0 sulfatée et se transforme en pentasaccharide $\Delta$Is-$IIa_{id}$-GlcA dès que le pH excède 8. Il est donc très important d'acidifier légèrement (pH entre 5 et 6) toute la fraction hexasaccharide. Le chromatogramme de la fraction hexasaccharide entière est donné sur la Figure 4.

La dernière phase consiste en une séparation semi-préparative sur colonne 25x2.1 cm remplie d'Hypersil BDS C18 (5 μm) greffée en CTA-SAX (Figure 5). Le contrôle des fractions est réalisé par CLHP (HPLC). La phase mobile utilisée en chromatographie semi-préparative étant une solution de chlorure de sodium, il est nécessaire de faire un dessalage final de l'échantillon. Celui-ci est réalisé en 2 étapes. La première étape, qui élimine 95 % du NaCl, consiste en une reconcentration des fractions contenant l'hexasaccharide isolé sur phase échangeuse d'anions Q-Sepharose High Flow (Pharmacia) (colonne 40 x 2,6 cm) en les faisant percoler dans la colonne après leur dilution à 1/10 dans l'eau. L'hexasaccharide est élué dans un volume minimal (environ 50 ml) par une solution de $NaClO_4$ 1,5N pour l'obtention d'une solution de perchlorate d'hexasaccharide.

La deuxième étape de dessalage final est réalisée en injectant la solution de perchlorate d'hexasaccharide obtenu précédemment, sur une colonne Sephadex G10 (100 x 7 cm). Le suivi est réalisé par détection UV à 232 nm et par un conductimètre qui permet de détecter le sel.

Il peut s'avérer nécessaire de réitérer cette opération si la qualité de la séparation entre l'hexasaccharide et le perchlorate est insuffisante. La solution d'hexasaccharide est ensuite lyophilisée. 108 mg de l'hexasaccharide $\Delta$Is-$IIa_{id}$-$\underline{IIs}_{glu}$ sous forme de sel de sodium sont ainsi obtenus. La pureté HPLC est de 92 % (Figure 6).

**Tests d'activité biologique Heparanase :**

*L'évaluation de l'Hexa Iso ATIII relativement à sa capacité à inhiber les héparanases a été effectuée comme suit :*

Du sulfate d'héparine/héparane radiomarqué (HS) est dégradé par des héparanase, produisant des fragments de HS de bas poids moléculaire qui peuvent être mesurés par chromatographie par perméation de gel (FPLC) et comptage des fractions collectées par scintillation liquide.

L'héparine non fractionnée (sel de sodium) de muqueuse intestinale de porc (grade Ia, 183 USP/mg) a été obtenue de Sigma Biochemicals (Deisenhofen, Allemagne).

L'héparitinase (HP lyase; (EC 4.2.2.8)) a été obtenue de Seigaku (Tokyo, Japon).

TSK 4000 provient de Toso Haas et les colonnes Sepharose Q équipées avec des pré-colonnes ont été obtenues de Pharmacia/LKB (Freiburg, Allemagne).

Une lignée cellulaire de fibroblastes d'utérus a été utilisée pour préparer du sulfate d'héparane (protéoglycane) marqué au 35-S par marquage métabolique. Il a été montré que cette lignée cellulaire produit des quantités relativement importantes de différents protéoglycanes de sulfate d'héparane (HS-PG = Heparan sulfate proteoglycans), tels que les syndécanes et glypicane (Drzeniek et al., Blood 93 :2884-2897, 1999).

Le marquage est réalisé par incubation des cellules avec une densité cellulaire d'approximativement 1 x $10^6$ cellules/ ml en présence de 35-S-sulfate à 33 μCi/ml dans le milieu de culture tissulaire pendant 24 heures. Les sumageants sont ensuite collectés et un inhibiteur de protéase PMSF (phenylmethylsulfonyl fluoride = fluorure de phénylméthylsulfonyle) (1 mmol/L) est ajouté. Les HS-PG sont purifiés par chromatographie d'échange d'anions sur Sepharose Q, l'élimination des sulfate de chondroïtine et sulfate de dermatane (protéoglycanes) n'étant pas nécessaire, puisque l'échantillon contient une quantité relativement importante de protéoglycanes de sulfate d'héparane, et aussi en raison de la spécificité de l'enzyme héparanase.

L'héparanase a été isolée de leucocytes sanguins humains (human peripheral blood leukocytes = PBL, buffy coats) et enrichie en cellules polymorphonucléaires (PMN = polymorphonuclear cells) par des procédures à gradient de ficoll. La concentration des PMN isolées est ajustée à 2,5 x $10^7$ cellules/ml et incubées pendant 1 heure à 4°C. Les surnageants contenant l'héparanase sont ensuite collectés, le pH est ajusté à 6,2 (20 mM de tampon citrate-phosphate) et sont soit utilisés immédiatement, soit stockés congelés dans des aliquotes à -20°C.

200 μl de sulfate d'héparane marqué au 35-S (protéoglycanes) ajustés à environ 2200 cpm/ml (cpm = coups par minute (counts per minute)) sont incubés à 37°C pendant 18 heures avec 1 μl de surnageant PMN contenant l'héparanase. 200 μl du mélange précédemment obtenu sont échantillonnés sur une colonne de chromatographie par perméation de gel TSK 4000 (FPLC), les fractions sont collectées et analysées par mesurage de scintillation liquide.

La dégradation a été mesurée selon la formule suivante :

% dégradation = [[Σ comptages (cpm) fract. 20-33 (HEP) - Σ comptages (cpm) fract. 20-33 (CONT)] / [comptages totaux (cpm) fract. 12-33 (CONT)]] x 100

Pour exemple, le pourcentage de dégradation est calculé comme suit: la somme des comptages (cpm) dans les fractions 20-33 de l'échantillon après traitement par l'héparanase soustrait du comptage du bruit de fond (cpm) (fractions 20-33) de l'échantillon de contrôle, est divisé par les comptages totaux (fractions 12-33) appliqués à la colonne. Des facteurs de correction ont été utilisés pour normaliser les comptages totaux de différentes chromatographies à 2200 comptages/cpm. Les résultats sont donnés en pourcentage de dégradation. Dans les tests d'inhibition, la dégradation de l'échantillon de contrôle (avec héparanase) a été fixée à 100 % (dégradation), et les valeurs de % d'inhibition ont été calculés sur cette base. Une correction pour l'activité sulfatase n'est pas nécessaire, puisque aucune activité sulfatase n'a pu être détectée.

Les inhibiteurs de l'héparanase suivants : héparine non fractionnée (UF-H) et Hexa Iso ATIII ont été testés via le protocole décrit ci-dessus à trois concentrations différentes. La comparaison a été effectuée sur une base de masse. Les données sont exprimées en pourcentage d'inhibition de l'activité héparanase.

**Résultats**

Premièrement, le test héparanase a été optimisé pour les besoins de cette étude. Pour des raisons pratiques, le temps d'incubation dans le test de dégradation a été établi à 18 heures. Selon l'efficacité de marquage et le contenu en sulfate d'héparane (protéoglycanes), le comptage total de sulfate d'héparane (protéoglycanes) a été fixé à environ 2200 cpm par échantillon, pour permettre d'effectuer tous les tests avec un lot de sulfate d'héparane (protéoglycane). La figure 1a présente une chromatographie par perméation de gel sur TSK 4000 d'un échantillon natif. La figure 1b présente le déplacement de la distribution moléculaire de l'échantillon induite par l'héparanase. La quantité d'héparanase permettant une dégradation d'environ 80 % de protéoglycane de sulfate d'héparane est ensuite déterminée (l'échantillon contenant approximativement 35 % de protéoglycanes de sulfate d'héparane et environ 65 % de protéoglycanes de sulfate de chondroïtine-/dermatane). Par conséquent, une dégradation dans un domaine de 10-80 % est relativement linéaire et est acceptable pour déterminer l'effet des inhibiteurs. La figure 1c présente l'effet d'héparine non fractionnée à 1 μg/ml (UFH =UnFractionned Heparin) sur l'activité héparanase avec une inhibition de 97,3 %.

Après avoir déterminé les conditions du test, l'effet d'héparine non-fractionnée (UFH) issue de muqueuse d'intestin de porc a été mesuré. La figure 2 présente une inhibition dépendante de la dose. A une concentration de 1 μg/ml en héparine non fractionnée (UFH) (concentration finale), une inhibition presque complète de l'activité héparanase a été observée. La figure 7 présente l'inhibition dépendante de la dose de l'Hexa Iso ATIII. Il peut être conclu sur la base de ces données que l'Hexa Iso ATIII présente une forte activité inhibitrice de l'héparanase.

C.R. Parish, et al., Biochim. Biophys. Acta 1471 (2001) 99-108

M. Bartlett et al., Immunol. Cell Biol. 73 (1995) 113-124

I. Vlodavsky et al., IMAJ 2 (2000) 37-45

Y. Matzner, et al., J. Clin. Invest. 76 (1985), 1306-1313

Z. Drzeniek, et al., Blood (1999) 2884-2897

D'autres fractions riches en oligosaccharides peuvent être isolées du produit de dégradation de l'héparine par l'héparinase I. Ainsi, dans le cas des hexasaccharides, une seule purification chromatographique CTA-SAX est suffisante. Cette méthode utilise une colonne Hypersil BDS (250 x 20 mm ) particules 5 μm, sur laquelle ont été greffées des chaînes cétyl triméthyl ammonium par percolation d'une solution d'hydrogénophosphate de cétyl triméthyl ammonium 1 mM dans un mélange eau-méthanol (68-32) v/v à 45°C à 2 ml/min pendant 4 heures.

La séparation est effectuée à température ambiante. Un gradient d'élution est utilisé : le solvant A est de l'eau mise à pH 2,5 par ajout de HCl. Le solvant B est une solution de NaCl 2N ajustée à pH 2,5.

| Temps (min) | Solvant A | Solvant B | Débit (ml/min) |
|---|---|---|---|
| 0 | 60 | 40 | 10 |
| 44 | 0 | 100 | 10 |

La détection est dans l'UV à 232 nm. On peut injecter 100 mg de fraction hexasaccharide à chaque séparation.

La purification des fractions octasaccharides et décasaccharides est plus complexe que celle des fractions hexasaccharides. Elle nécessite en général une purification supplémentaire sur colonne IonPac ®AS11 (250x20mm) (Dionex ). La séparation est effectuée à température ambiante. Un gradient d'élution est utilisé. Le solvant A est de l'eau mise à pH 3 par ajout d'acide perchlorique. Le solvant B est une solution de $NaClO_4$ 1M ajusté à pH3.

| Temps (min) | Solvant A | Solvant B | Débit (ml/min) |
|---|---|---|---|
| 0 | 99 | 1 | 20 |
| 80 | 40 | 60 | 20 |

La figure 8 permet d'identifier les produits isolés des fractions riches en oligosaccharides, pour chacune des fractions hexasaccharide, octasaccharide et décasaccharide isolées par GPC.

**Evaluation de l'activité des inhibiteurs d'héparanases dans un système enzymatique**

L'activité de l'héparanase est mise en évidence par sa capacité à dégrader le fondaparinux. La concentration de fondaparinux est déterminée grâce à son activité anti-facteur Xa.

***A. Matériel et méthodes***

L'héparanase est produit par Sanofi-Synthélabo (Labège, France).

Les réactifs de dosages du facteur Xa sont commercialisés par Chromogénix (Montpellier, France).

Des concentrations croissantes d'un composé selon l'invention, inhibiteur d'héparanases (dilutions variables : de 1nM à 10 µM) sont mélangées à une concentration fixe d'héparanase (pour chaque lot, des expériences préliminaires permettent de déterminer l'activité enzymatique suffisante à la dégradation de 0,45µg/ml de fondaparinux ajouté). Après 5 minutes à 37°C le mélange est mis en présence du fondaparinux et laissé 1 heure à 37°C. La réaction est arrêtée par chauffage à 95 °C pendant 5 minutes. La concentration de fondaparinux résiduelle est enfin mesurée par addition de facteur Xa et de son substrat chromogène spécifique (Ref. S2222).

**Les différents mélanges sont effectués selon la procédure qui suit :**

**a) Mélange réactionnel**

On mélange 50 µl de tampon acétate de sodium (0.2 M, pH 4.2), avec 50 µl de fondaparinux (0.45 µg/ml) et 59 µl d'une solution d'héparanase). On incube 1 heure à 37 °C, puis 5 minutes à 95°C. On passe ainsi du pH 4.2 à pH 7. On mélange ensuite 100 µl du mélange réactionnel avec 50 µl de tampon Tris 50 mM, NaCl 175 mM EDTA 75 mM, pH 14.

L'activité anti-facteur Xa du fondaparinux est mesurée de la façon qui suit :

**b) Dosage de l'activité anti-facteur Xa du fondaparinux**

On mélange 100 µl de la solution obtenue à l'étape a) avec 100 µl d'AT (0.5 µ/ml). On maintient pendant 2 minutes à 37°C et on ajoute ensuite 100 µl de facteur Xa (7nkat/ml). On maintient pendant 2 minutes à 37°C et on ajoute ensuite 100 µl de substrat chromogénique (Ref.: S2222) (1 mM). On maintient pendant 2 minutes à 37 °C et on ajoute ensuite 100 µl d'acide acétique (50 %).

La lecture de la densité optique s'effectue à 405 nm.

Un pourcentage d'inhibition est déterminé par rapport au contrôle sans inhibiteur. Une courbe des pourcentages d'inhibition permet de calculer une $IC_{50}$.

***B. Résultats***

| Produit | Structure | Concentration (M) | % d'inhibition |
|---|---|---|---|
| Hexa Iso ATIII | ΔIs-IIa-IIs | 3.00E-5 | 48.5 |
| (Ia) | ΔIs-IIa-IIs | 1.00E-4 | 53.8 |
| (Ib) | ΔIs-Is-IIa-IIs | 1.00E-5 | 59.9 |
| (Ic) | ΔIs-Is-IIa-IIs | 3.00E-6 | 59.2 |
| (Id) | ΔIs-Is-Ia-IIs | 3.00E-5 | 53.9 |
| (Ie) | ΔIs-Is-Is-IIs | 3.00E-5 | 59.1 |
| (If) | ΔIs-Is-Is-IIa-IIs | 3.00E-6 | 58.4 |
| (Ig) | ΔIs-Is-Is-IIa-IIs | 1.00E-4 | 55.8 |
| (Ih) | ΔIs-Is-Is-Ia-IIs | 3.00E-5 | 55.5 |
| (Ij) | ΔIs-Is-Is-Is-IIs | 3.00E-5 | 48.4 |
| (Im) | ΔIs-Ia-IIs | 3.00E-5 | 54.1 |
| Fraction hexasaccharide | | 3.00E-5 | 55.8 |
| Fraction octasaccharide | | 3.00E-6 | 50.7 |
| Fraction décasaccharide | | 3.00E-6 | 55.6 |
| Brut après dépolymérisation | | 3.00E-6 | 53.0 |

**Revendications**

1. Procédé de dépolymérisation d'un polysaccharide à propriété anti-thrombotique, pour l'obtention d'un produit ayant des propriétés anticancéreuses, **caractérisé en ce qu'**il comprend une étape dans laquelle le polysaccharide est dépolymérisé par l'héparinase I jusqu'à ce que son activité anti-thrombotique, due notamment à l'inhibition des facteurs Xa et IIa, soit essentiellement éteinte (<35 UI/mg) et en que la dépolymérisation est conduite à une température comprise entre 10 et 20°C.

2. Procédé selon la revendication 1, **caractérisé en ce que** le polysaccharide est une héparine.

3. Procédé selon la revendication 1 ou la revendication 2, **caractérisé en ce que** la dépolymérisation est poursuivie jusqu'à ce que le mélange comprenne une fraction hexasaccharidique essentiellement exempte d'hexasaccharides sulfatés $\Delta Is\text{-}Is_{id}\text{-}Is_{id}$ et $\Delta Is\text{-}Is_{id}\text{-}IIs_{glu}$.

4. Procédé selon l'une quelconque des revendications 1 à 3, **caractérisé en ce que** la dépolymérisation est poursuivie jusqu'à atteindre une masse moléculaire moyenne inférieure à 5000 Da.

5. Procédé selon l'une quelconque des revendications 1 à 3, **caractérisé en ce que** la dépolymérisation est poursuivie jusqu'à atteindre une masse moléculaire moyenne inférieure à 3000 Da.

6. Procédé selon la revendication 1 ou la revendication 2, **caractérisé en ce qu'**il comprend en outre une étape dans laquelle le produit de dépolymérisation du polysaccharide est purifié par chromatographie par perméation de gel à un pH inférieur à 8 et supérieur à 5.

**7.** Procédé selon la revendication 6, **caractérisé en ce qu'**il comprend en outre une étape de purification par chromatographie liquide haute performance (HPLC) dans laquelle une phase stationnaire est une phase inverse greffée (i) en C18 et (ii) en cétyl triméthyl ammonium (CTA SAX).

**8.** Procédé selon la revendication 7, **caractérisé en ce qu'**il comprend en outre une étape de dessalage.

**9.** Procédé selon la revendication 8, dans laquelle l'étape de dessalage comprend l'utilisation d'une phase mobile contenant un électrolyte en solution aqueuse, essentiellement transparent entre 200 et 250 nm.

**10.** Procédé selon la revendication 9, **caractérisé en ce que** l'électrolyte est choisi parmi les perchlorates, méthanesulfonates ou phosphates de métaux alcalins tels que Na.

**11.** Procédé selon la revendication 10, **caractérisé en ce que** le dessalage est effectué à l'aide d'une résine échangeuse d'anions, par exemple de type Sepharose Q ®.

**12.** Procédé selon l'une quelconque des revendications 8 à 11, **caractérisé en ce qu'**il comprend une seconde étape de dessalage à l'aide d'un gel d'exclusion moléculaire, en particulier de type Sephadex G10 ®.

**13.** Produit obtenu par le procédé selon l'une quelconque des revendications 1 à 12.

**14.** Produit de formule générale (I)

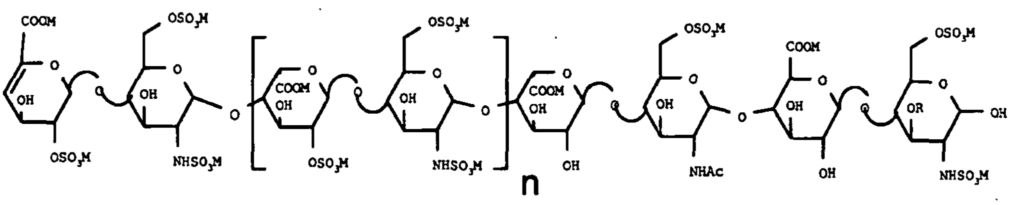

Δ Is $Is_{idu}$ $IIa_{idu}$ $IIs_{\underline{glu}}$ / $IIs_{glu}$

n = 0 à 5

dans laquelle:

R est choisi parmi H et $SO_3M$, et
M est choisi parmi H, Li, Na et K ;

à l'exception du produit pour lequel n = 0, R = SO3M et M = Na.

**15.** Produit selon la revendication 14, **caractérisé en ce que** M est choisi parmi Li, Na et K.

**16.** Produit selon la revendication 14 ou la revendication 15, **caractérisé en ce que** n = 0.

**17.** Produit selon la revendication 15 ou la revendication 16, **caractérisé en ce que** M = Na.

**18.** Produit selon la revendication 17, de formule (Ia) suivante :

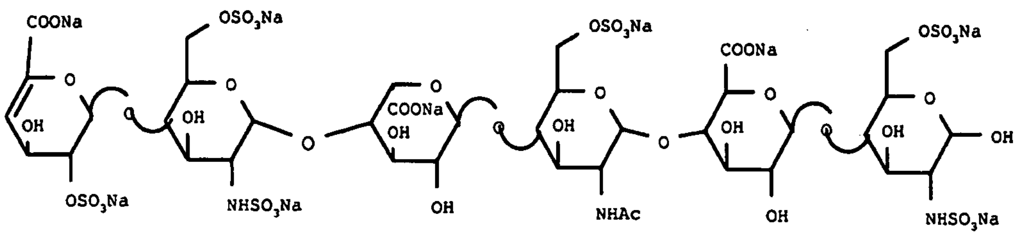

Δ Is $IIa_{idu}$ $IIs_{glu}$

**19.** Produit selon la revendication 14, de formule (Ib) suivante :

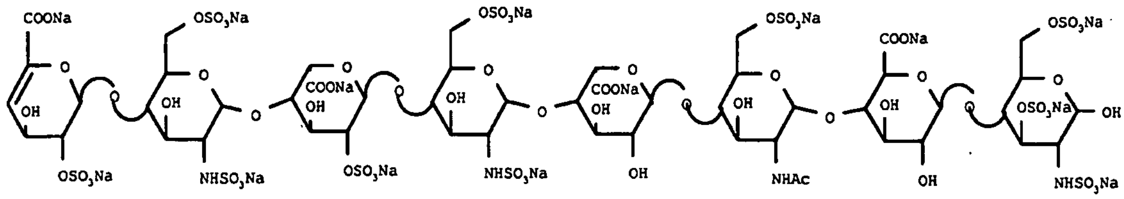

ΔIs Is IIa IIs

**20.** Produit selon la revendication 14, de formule (Ic) suivante :

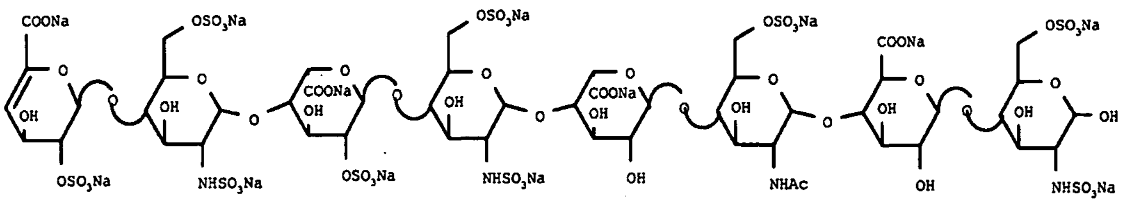

ΔIs Is IIa IIs

**21.** Produit de formule (Id) suivante :

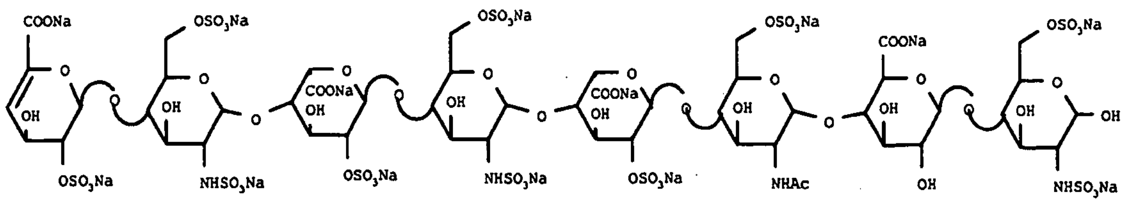

ΔIs Is Ia IIs

**22.** Produit de formule (Ie) suivante :

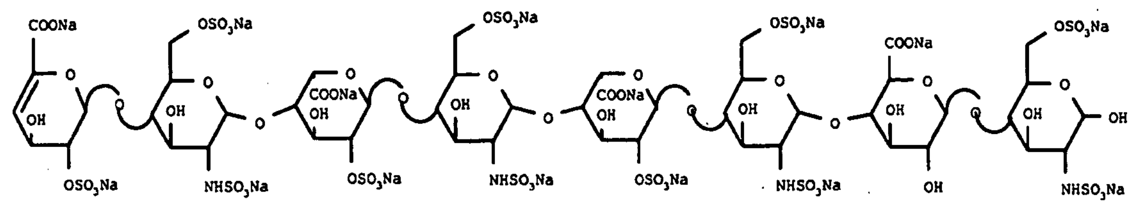

ΔIs Is Is IIs

**23.** Produit selon la revendication 14, de formule (If) suivante :

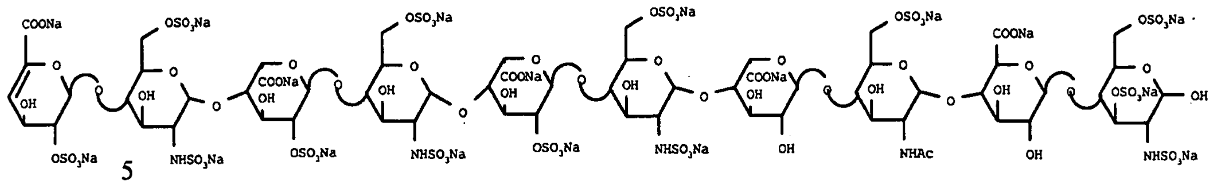

ΔIs Is Is IIa IIs

**24.** Produit selon la revendication 14, de formule (Ig) suivante :

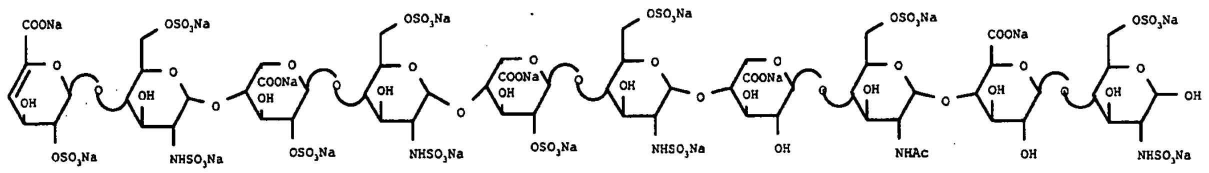

ΔIs Is Is IIa IIs

**25.** Produit de formule (Ih) suivante :

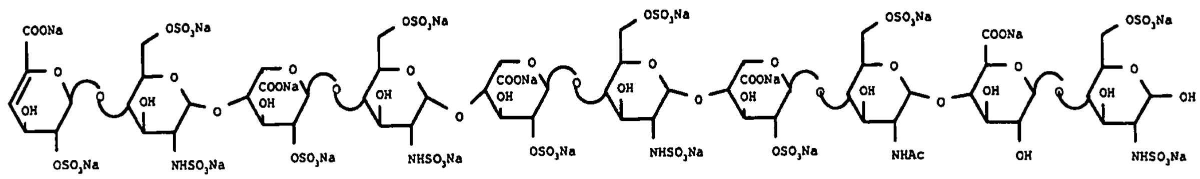

ΔIs Is Is Ia IIs

**26.** Produit de formule (Ij) suivante :

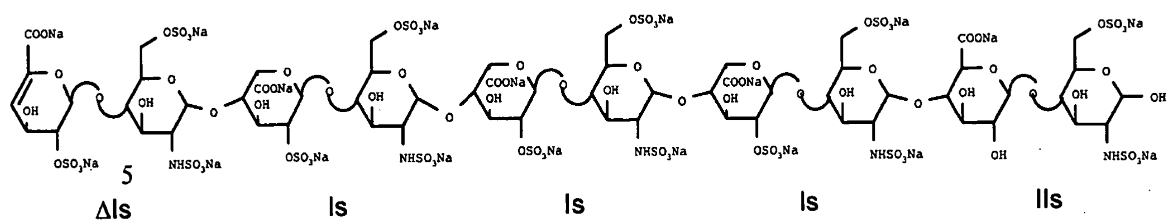

ΔIs Is Is Is IIs

**27.** Produit selon la revendication 14, de formule (Ik) suivante :

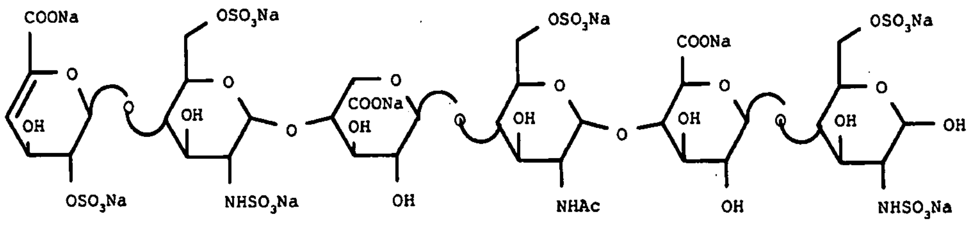

ΔIs IIa IIs

**28.** Produit de formule (Im) suivante :

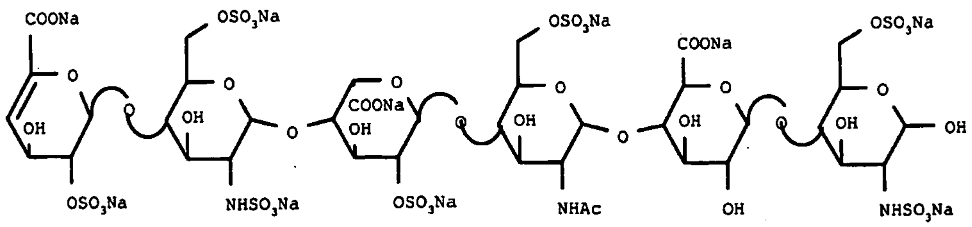

ΔIs Ia IIs

**29.** Utilisation d'un produit de formule générale (I)

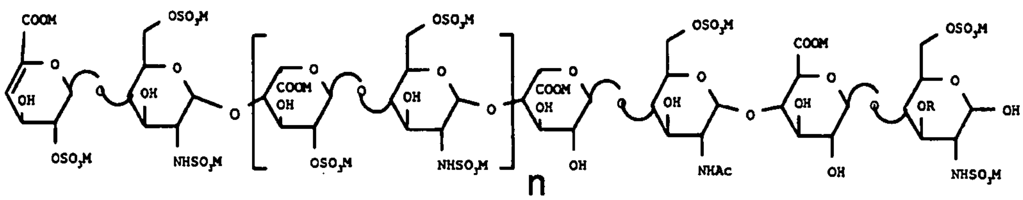

Δ Is $Is_{idu}$ $IIa_{idu}$ $IIs_{\underline{glu}}$ / $IIs_{glu}$

n = 0 à 5

dans laquelle :

R est choisi parmi H et $SO_3M$, et
M est choisi parmi H, Li, Na et K ;

en tant qu'inhibiteur de l'héparanase.

**30.** Utilisation d'un produit de formule générale (I) selon la revendication 29, **caractérisée en ce qu'**il s'agit de :

A D

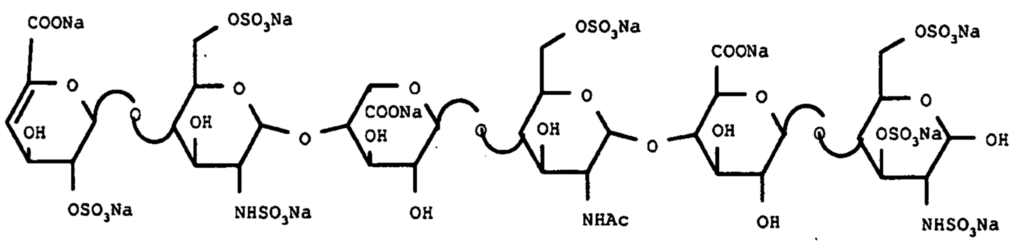

Δ Is IIa$_{idu}$ IIs$_{glu}$

**31.** Utilisation d'un produit de formule Ia, Ib, Ic, Id, Ie, If, Ig, Ih, Ij, Ik, ou Im en tant qu'inhibiteur de l'héparanase.

**32.** Utilisation d'un produit selon l'une quelconque des revendications 29 à 31, pour moduler une prolifération cellulaire.

**33.** Utilisation d'un produit selon la revendication 32, **caractérisée en ce que** la prolifération cellulaire est liée au cancer.

**34.** Utilisation d'un produit selon la revendication 33, **caractérisée en ce que** la prolifération cellulaire est liée à un processus métastatique.

**35.** Utilisation d'un produit selon la revendication 33 ou la revendication 34, en tant qu'agent cytostatique.

**36.** Utilisation d'un produit selon la revendication 34 ou la revendication 35, **caractérisée en ce qu'**elle est effectuée à un stade précoce de la maladie.

**37.** Utilisation d'un produit selon l'une quelconque des revendications 35 à 36, en combinaison avec un second produit anticancéreux.

**38.** Utilisation d'un produit selon la revendication 37, **caractérisée en ce que** le second produit anticancéreux est cytotoxique.

**39.** Utilisation d'un produit selon la revendication 38, **caractérisée en ce que** le second produit anticancéreux est choisi parmi des dérivés du platine tels que le cisplatine ou l'oxaliplatine, des taxoïdes tels que le docétaxel ou le paclitaxel, des dérivés de bases puriques ou pyrimidiques telles que le 5-FU, la capécitabine ou la gemcitabine, des vincas tels que la vincristine ou la vinblastine, des moutardes, des hétérocycles aromatiques condensés tels que la staurosporine, l'éllipticine ou les camptothécines telles que l'irinotécan, le topotécan, des combrétastatines telles que la CA4P, des dérivés de la colchicine, tels que le phosphate de colchinol.

**40.** Utilisation d'un produit selon la revendication 39, **caractérisée en ce que** le second produit anticancéreux est le docétaxel, l'oxaliplatine ou l'irinotécan.

**41.** Utilisation d'un produit selon l'une quelconque des revendications 32 à 40, **caractérisée en ce que** le cancer est un cancer du sein, du poumon, de la prostate, du colon ou du pancréas.

## Claims

**1.** Method for depolymerizing a polysaccharide with antithrombotic properties, in order to obtain a product having anticancer properties, **characterized in that** it comprises a step in which the polysaccharide is depolymerized with heparinase I until its antithrombotic activity, due in particular to the inhibition of factors Xa and IIa, is essentially extinguished (< 35 IU/mg), and **in that** the depolymerization is carried out at a temperature of between 10 and 20°C.

**2.** Method according to Claim 1, **characterized in that** the polysaccharide is heparin.

**3.** Method according to Claim 1 or Claim 2, **characterized in that** the depolymerization is pursued until the mixture comprises a hexasaccharide fraction essentially free of sulphated hexasaccharides $\Delta Is\text{-}Is_{id}\text{-}Is_{id}$ and $\Delta Is\text{-}Is_{id}\text{-}IIs_{glu}$.

**4.** Method according to any one of Claims 1 to 3, **characterized in that** the depolymerization is pursued until an average molecular mass of less 5000 Da is attained.

**5.** Method according to any one of Claims 1 to 3, **characterized in that** the depolymerization is pursued until an average molecular mass of less 3000 Da is attained.

**6.** Method according to Claim 1 or Claim 2, **characterized in that** it also comprises a step in which the product of depolymerization of the polysaccharide is purified by gel permeation chromatography at a pH below 8 and above 5.

**7.** Method according to Claim 6, **characterized in that** it also comprises a step of purification by high performance liquid chromatography (HPLC) in which a stationary phase is a reverse phase which is (i) C18-grafted and (ii) grafted with cetyl trimethylammonium (CTA-SAX).

**8.** Method according to Claim 7, **characterized in that** it also comprises a desalification step.

**9.** Method according to Claim 8, in which the desalification step comprises the use of a mobile phase containing an electrolyte in aqueous solution, essentially transparent between 200 and 250 nm.

**10.** Method according to Claim 9, **characterized in that** the electrolyte is chosen from perchlorates, methanesulphonates or phosphates of alkali metals such as Na.

**11.** Method according to Claim 10, **characterized in that** the desalification is carried out using an anion exchange resin, for example of the Sepharose Q® type.

**12.** Method according to any one of Claims 8 to 11, **characterized in that** it comprises a second desalification step using a molecular exclusion gel, in particular of the Sephadex G10® type.

**13.** Product obtained by the method according to any one of Claims 1 to 12.

**14.** Product of formula (I)

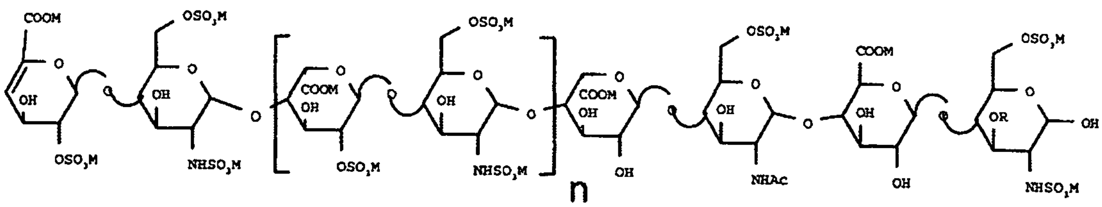

$\Delta$ Is    $Is_{idu}$    $IIa_{idu}$    $IIs_{glu}$ / $IIs_{glu}$

n = 0 to 5

in which

R is chosen from H and $SO_3M$, and
M is chosen from H, Li, Na and K;

with the exception of the product for which n = 0, R = $SO_3M$ and M = Na.

**15.** Product according to Claim 14, **characterized in that** M is chosen from Li, Na and K.

**16.** Product according to Claim 14 or Claim 15, **characterized in that** n = 0.

**17.** Product according to Claim 15 or Claim 16, **characterized in that** M = Na.

**18.** Product according to Claim 17, of formula (Ia) below:

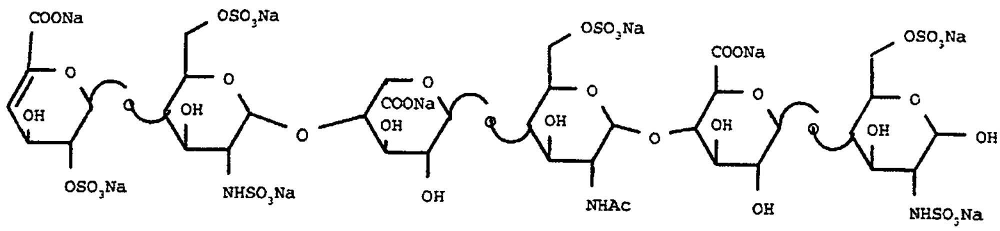

Δ Is $IIa_{idu}$ $IIs_{glu}$

**19.** Product according to Claim 14, of formula (Ib) below:

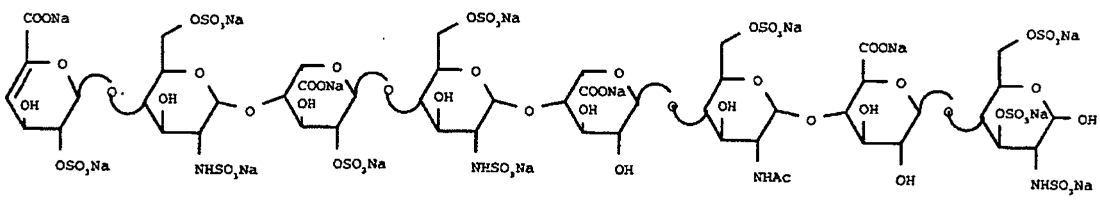

ΔIs Is IIa IIs

**20.** Product according to Claim 14, of formula (Ic) below:

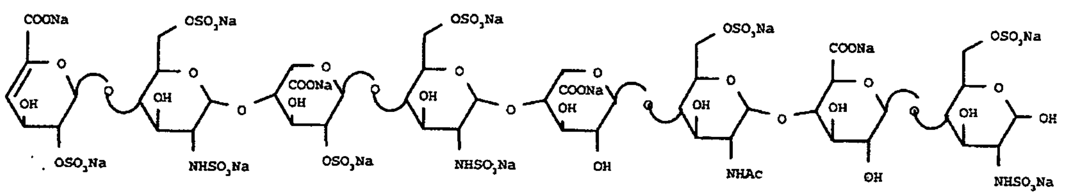

ΔIs Is IIa IIs

**21.** Product of formula (Id) below:

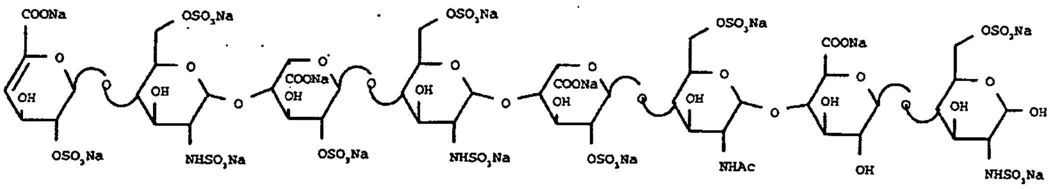

ΔIs Is Ia IIs

**22.** Product of formula (Ie) below:

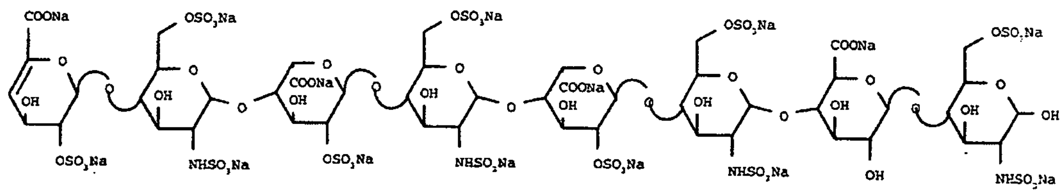

ΔIs Is Is IIs

**23.** Product according to Claim 14, of formula (If) below:

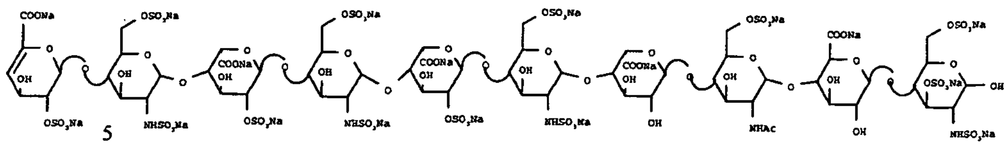

ΔIs Is Is IIa IIs

**24.** Product according to Claim 14, of formula (Ig) below:

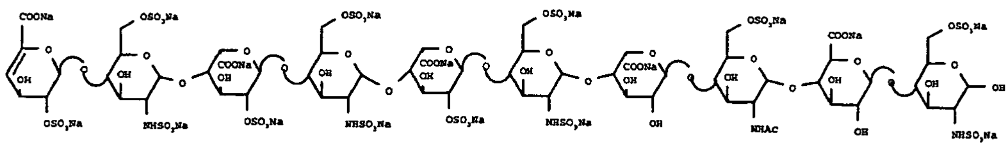

ΔIs Is Is IIa IIs

**25.** Product of formula (Ih) below:

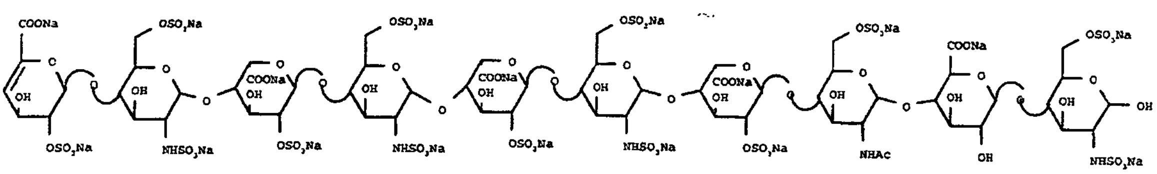

ΔIs Is Is Ia IIs

**26.** Product of formula (Ij) below:

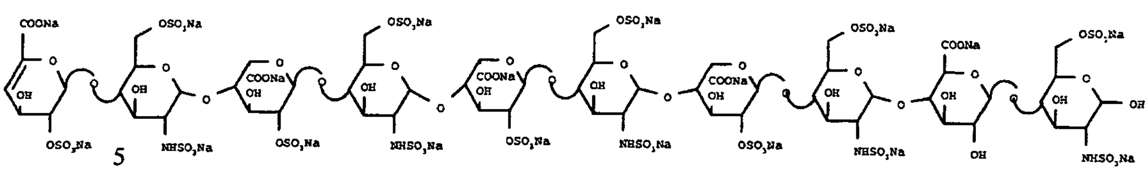

ΔIs Is Is Is IIs

**27.** Product according to Claim 14, of formula (Ik) below:

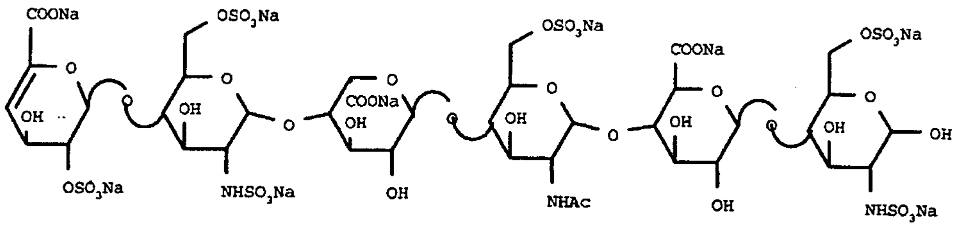

ΔIs IIa IIs

**28.** Product of formula (Im) below:

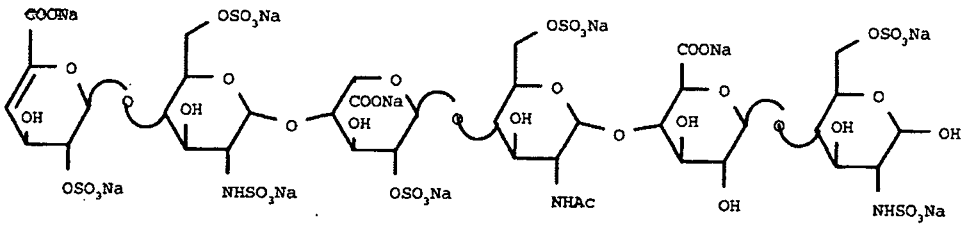

ΔIs Ia IIs

**29.** Use of a product of formula (I)

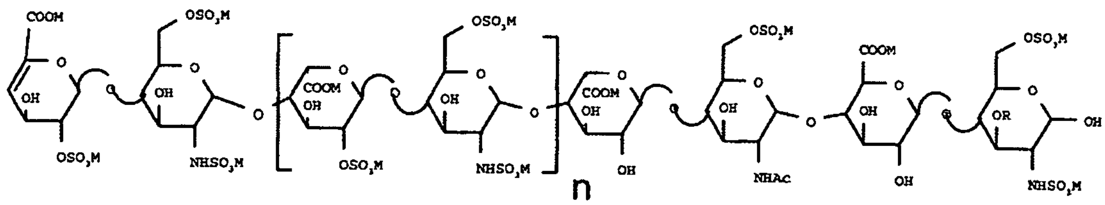

Δ Is $Is_{idu}$ $IIa_{idu}$ $IIs_{glu}$ / $IIs_{glu}$

n = 0 to 5

in which

R is chosen from H and $SO_3M$, and
M is chosen from H, Li, Na and K;

as a heparanase inhibitor.

**30.** Use of a product of formula (I) according to Claim 29, **characterized in that** it is:

A D

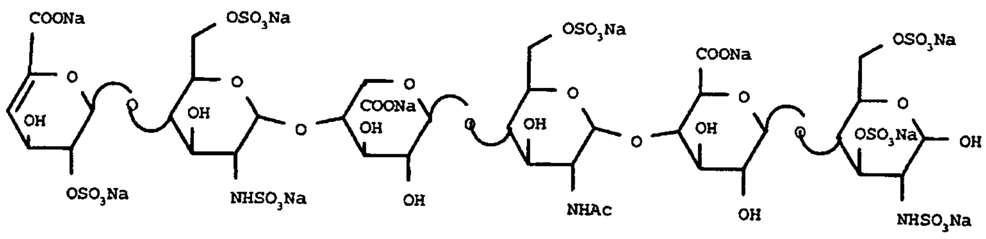

Δ Is $IIa_{idu}$ $IIs_{glu}$

**31.** Use of a product of formula Ia, Ib, Ic, Id, Ie, If, Ig, Ih, Ij, Ik or Im, as a heparanase inhibitor.

**32.** Use of a product according to any one of Claims 29 to 31, for modulating cell proliferation.

**33.** Use of a product according to Claim 32, **characterized in that** the cell proliferation is related to cancer.

**34.** Use of a product according to Claim 33, **characterized in that** the cell proliferation is related to a metastatic process.

**35.** Use of a product according to Claim 33 or Claim 34, as a cytostatic agent.

**36.** Use of a product according to Claim 34 or Claim 35, **characterized in that** it is effected at an early stage of the disease.

**37.** Use of a product according to either one of Claims 35 and 36, in combination with a second anticancer product.

**38.** Use of a product according to Claim 37, **characterized in that** the second anticancer product is cytotoxic.

**39.** Use of a product according to Claim 38, **characterized in that** the second anticancer product is chosen from platinum derivatives such as cisplatin or oxaliplatin, taxoids such as docetaxel or paclitaxel, purine base or pyrimidine base derivatives such as 5-FU, capecitabine or gemcitabine, vincas such as vincristine or vinblastine, mustards, condensed aromatic heterocycles such as staurosporine, ellipticine or camptothecins such as irinotecan, topotecan, combretastatines such as CA4P, and colchicine derivatives such as colchinol phosphate.

**40.** Use of a product according to Claim 39, **characterized in that** the second anticancer product is docetaxel, oxaliplatin or irinotecan.

**41.** Use of a product according to any one of Claims 32 to 40, **characterized in that** the cancer is breast cancer, lung cancer, prostate cancer, colon cancer or pancreatic cancer.

**Patentansprüche**

**1.** Verfahren zur Depolymerisation eines antithrombotischen Polysaccharids zur Gewinnung eines Produkts mit krebshemmenden Eigenschaften, **dadurch gekennzeichnet, daß** das Verfahren einen Schritt umfaßt, bei dem das Polysaccharid mit Heparinase I so lange depolymerisiert wird, bis seine antithrombotische Wirkung, die insbesondere auf der Hemmung der Faktoren Xa und IIa beruht, im wesentlichen vergangen ist (<35 IU/mg), und **dadurch**, daß die Depolymerisation bei einer Temperatur zwischen 10 und 20°C durchgeführt wird.

**2.** Verfahren nach Anspruch 1, **dadurch gekennzeichnet, daß** es sich bei dem Polysaccharid um ein Heparin handelt.

**3.** Verfahren nach Anspruch 1 oder Anspruch 2, **dadurch gekennzeichnet, daß** die Depolymerisation so lange durchgeführt wird, bis die Mischung eine Hexasaccharidfraktion, die im wesentlichen frei von den sulfierten Hexasacchariden $\Delta Is\text{-}Is_{id}\text{-}Is_{id}$ und $\Delta Is\text{-}Is_{id}\text{-}IIs_{glu}$ frei ist, umfaßt.

**4.** Verfahren nach einem der Ansprüche 1 bis 3, **dadurch gekennzeichnet, daß** die Depolymerisation solange durchgeführt wird, bis man zu einem mittleren Molekulargewicht von unter 5000 Da gelangt.

**5.** Verfahren nach einem der Ansprüche 1 bis 3, **dadurch gekennzeichnet, daß** die Depolymerisation solange durchgeführt wird, bis man zu einem mittleren Molekulargewicht von unter 3000 Da gelangt.

**6.** Verfahren nach Anspruch 1 oder Anspruch 2, **dadurch gekenntzeichnet, daß** das Verfahren weiterhin einen Schritt umfaßt, bei dem das Produkt der Depolymerisation des Polysaccharids mittels Gelpermeationschromatographie bei einem pH-Wert von unterhalb 8 und oberhalb 5 aufgereinigt wird.

**7.** Verfahren nach Anspruch 6, **dadurch gekennzeichnet, daß** das Verfahren weiterhin einen Aufreinigungsschritt mittels Hochleistungsflüssigkeitschromatographie (HPLC) umfaßt, bei dem eine stationäre Phase eine Umkehrphase ist, die (i) mit C18 und (ii) mit Cetyltrimethylammonium (CTA-SAX) gepfropft ist.

**8.** Verfahren nach Anspruch 7, **dadurch gekennzeichnet, daß** es weiterhin einen Entsalzungssschritt umfaßt.

**9.** Verfahren nach Anspruch 8, bei dem der Entsalzungsschritt die Verwendung einer mobilen Phase enthaltend einen Elektrolyten in wäßriger Lösung, im wesentlichen transparent zwischen 100 und 250 nm, umfaßt.

**10.** Verfahren nach Anspruch 9, **dadurch gekennzeichnet, daß** der Elektrolyt aus der Reihe der Perchlorate, Methansulfonate oder Phosphate von Alkalimetallen wie Na stammt.

**11.** Verfahren nach Anspruch 10, **dadurch gekennzeichnet, daß** die Entsalzung mit Hilfe eines Anionenaustauscherharzes, zum Beispiel des Typs Sepharose Q ®, durchgeführt wird.

**12.** Verfahren nach einem der Ansprüche 8 bis 11, **dadurch gekennzeichnet, daß** es einen zweiten Entsalzungsschritt mit Hilfe eines Molekülausschlußgels, insbesondere des Typs Sephadex G10 ®, umfaßt.

**13.** Produkt, erhalten nach dem Verfahren nach einem der Ansprüche 1 bis 12.

**14.** Produkt der allgemeinen Formel (I)

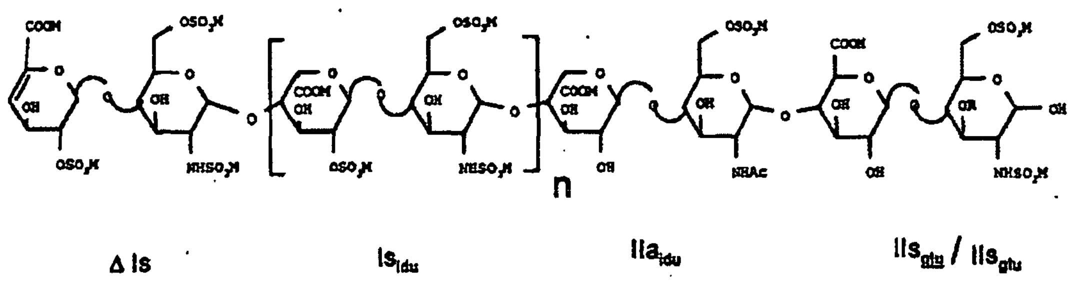

n = 0 bis 5

in der:

R aus der Reihe H und $SO_3M$ stammt, und
M aus der Reihe H, Li, Na und K stammt;

mit Ausnahme des Produkts, bei dem n = 0, R = S03M und M = Na.

**15.** Produkt nach Anspruch 14, **dadurch gekennzeichnet, daß** M aus der Reihe Li, Na und K stammt.

**16.** Produkt nach Anspruch 14 oder Anspruch 15, **dadurch gekennzeichnet, daß** n = 0.

**17.** Produkt nach Anspruch 15 oder Anspruch 16, **dadurch gekennzeichnet, daß** M = Na.

**18.** Produkt nach Anspruch 17 der folgenden Formel (Ia):

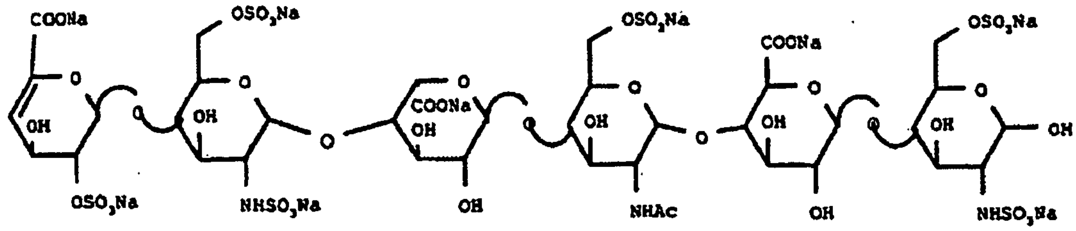

Δ Is IIa$_{idu}$ IIs$_{glu}$

**19.** Produkt nach Anspruch 14 der folgenden Formel (Ib):

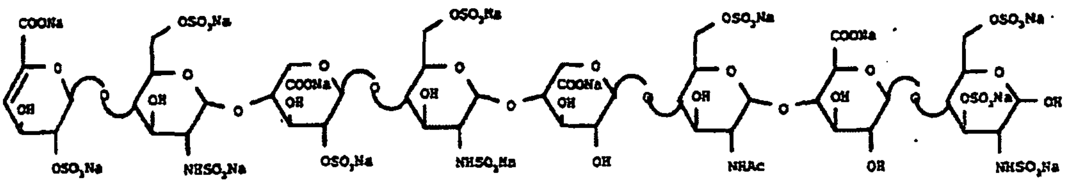

ΔIs Is IIa IIs

**20.** Produkt nach Anspruch 14 der folgenden Formel (Ic):

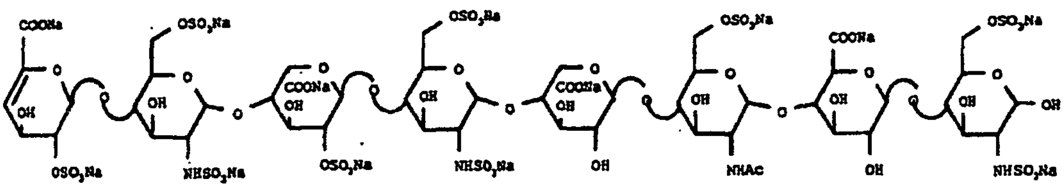

ΔIs Is IIa IIs

**21.** Produkt der folgenden Formel (Id):

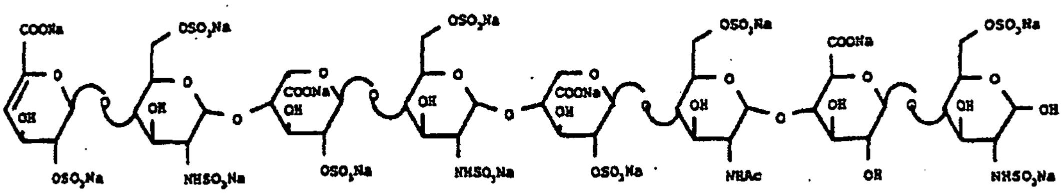

ΔIs Is Ia IIs

**22.** Produkt der folgenden Formel (Ie):

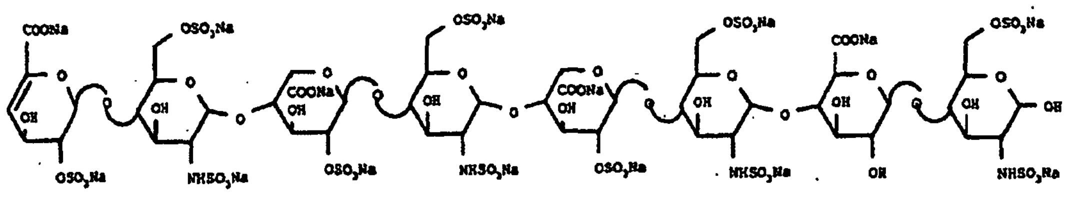

ΔIs Is Is IIs

**23.** Produkt nach Anspruch 14 der folgenden Formel (If) :

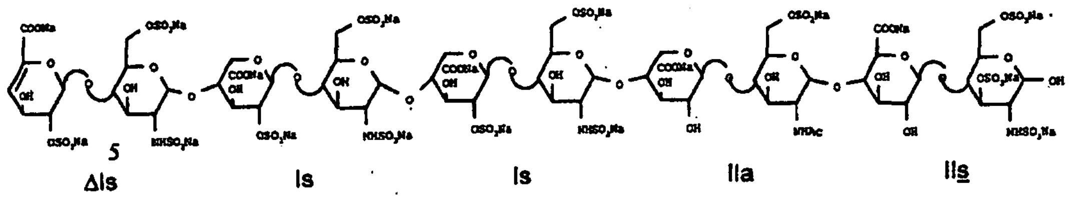

ΔIs Is Is IIa IIs

**24.** Produkt nach Anspruch 14 der folgenden Formel (Ig):

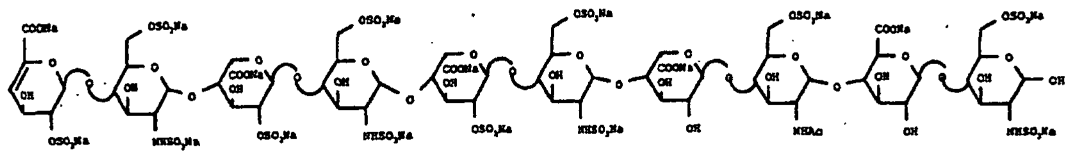

ΔIs Is Is IIa IIs

**25.** Produkt der folgenden Formel (Ih):

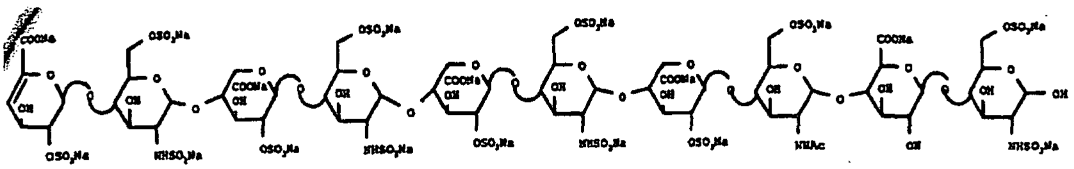

ΔIs Is Is Ia IIs

**26.** Produkt der folgenden Formel (Ij):

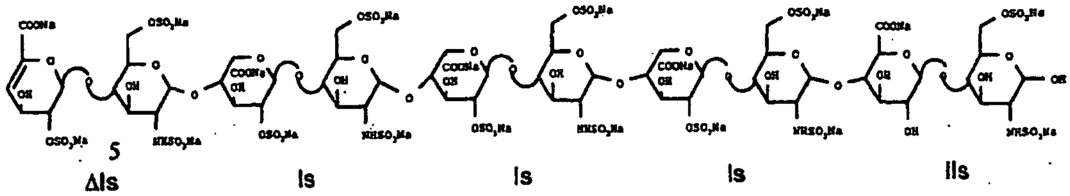

ΔIs Is Is Is IIs

**27.** Produkt nach Anspruch 14 der folgenden Formel (Ik) :

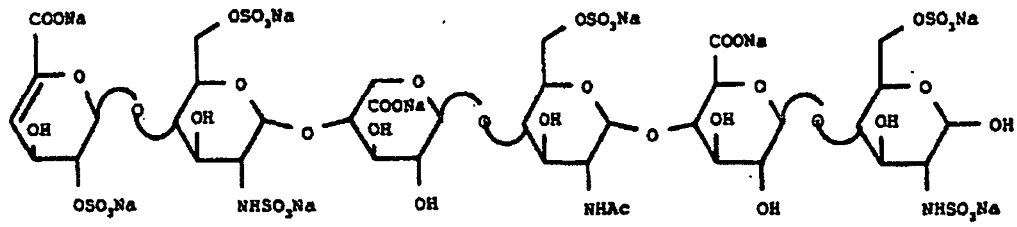

ΔIs IIa IIs

**28.** Produkt der folgenden Formel (Im):

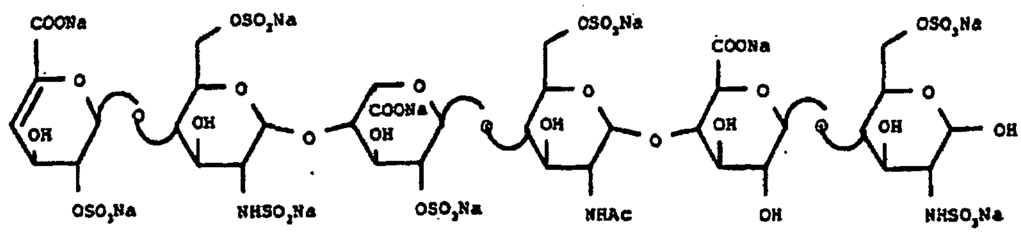

ΔIs Ia IIs

**29.** Verwendung eines Produkts der allgemeinen Formel (I)

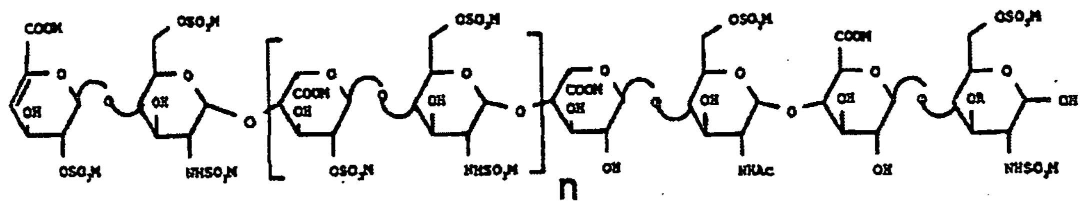

Δ Is $Is_{Idu}$ $IIa_{Idu}$ $IIs_{glu}$ / $IIs_{glu}$

n = 0 bis 5

in der:

R aus der Reihe H und $SO_3M$ stammt, und
M aus der Reihe H, Li, Na und K stammt;

als Heparinasehemmer.

**30.** Verwendung eines Produkts der allgemeinen Formel (I) nach Anspruch 29, **dadurch gekennzeichnet, daß** es sich um

A D

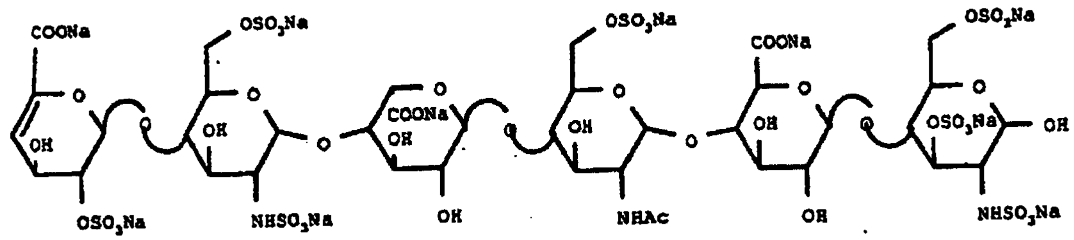

Δ Is IIa$_{idu}$ IIs$_{glu}$

handelt.

**31.** Verwendung eines Produkts der Formel Ia, Ib, Ic, Id, Ie, If, Ig, Ih, Ij, Ik oder Im als Heparinasehemmer.

**32.** Verwendung eines Produkts nach einem der Ansprüche 29 bis 31 zur Modulation der Zellproliferation.

**33.** Verwendung eines Produkts nach Anspruch 32, **dadurch gekennzeichnet, daß** die Zellproliferation mit Krebs assoziiert ist.

**34.** Verwendung eines Produkts nach Anspruch 33, **dadurch gekennzeichnet, daß** die Zellproliferation mit einer Metastasierung assoziiert ist.

**35.** Verwendung eines Produkts nach Anspruch 33 oder Anspruch 34 als Zytostatikum.

**36.** Verwendung eines Produkts nach Anspruch 34 oder Anspruch 35, **dadurch gekennzeichnet, daß** sie in einem Frühstadium der Krankheit erfolgt.

**37.** Verwendung eines Produkts nach einem der Ansprüche 35 bis 36 in Kombination mit einem zweiten krebshemmenden Mittel.

**38.** Verwendung eines Produkts nach Anspruch 37, **dadurch gekennzeichnet, daß** das zweite krebshemmende Produkt zytotoxisch ist.

**39.** Verwendung eines Produkts nach Anspruch 38, **dadurch gekennzeichnet, daß** das zweite krebshemmende Produkt aus der folgenden Reihe stammt: Platinderivate wie Cisplatin oder Oxaliplatin, Taxoide wie Docetaxel oder Paclitaxel, Derivate von Purin- oder Pyrimidinbasen wie 5-FU, Capecitabin oder Gemcitabin, Vincas wie Vincristin oder Vinblastin, Senfe, kondensierte aromatische Heterocyclen wie Staurosporin, Ellipticin oder Camptothecine wie Irinotecan, Topotecan, Combretastatine wie CA4P, Colchicinderivate wie Colchinolphosphat.

**40.** Verwendung eines Produkts nach Anspruch 39, **dadurch gekennzeichnet, daß** es sich bei dem zweiten krebshemmenden Produkt um Docetaxel, Oxaliplatin oder Irinotecan handelt.

**41.** Verwendung eines Produkts nach einem der Ansprüche 32 bis 40, **dadurch gekennzeichnet, daß** es sich bei dem Krebs um Brustkrebs, Lungenkrebs, Prostatakrebs, Dickdarmkrebs oder Pancreaskrebs handelt.

**Suivi GPC de la dépolymérisation de l'héparine par l'héparinase 1**

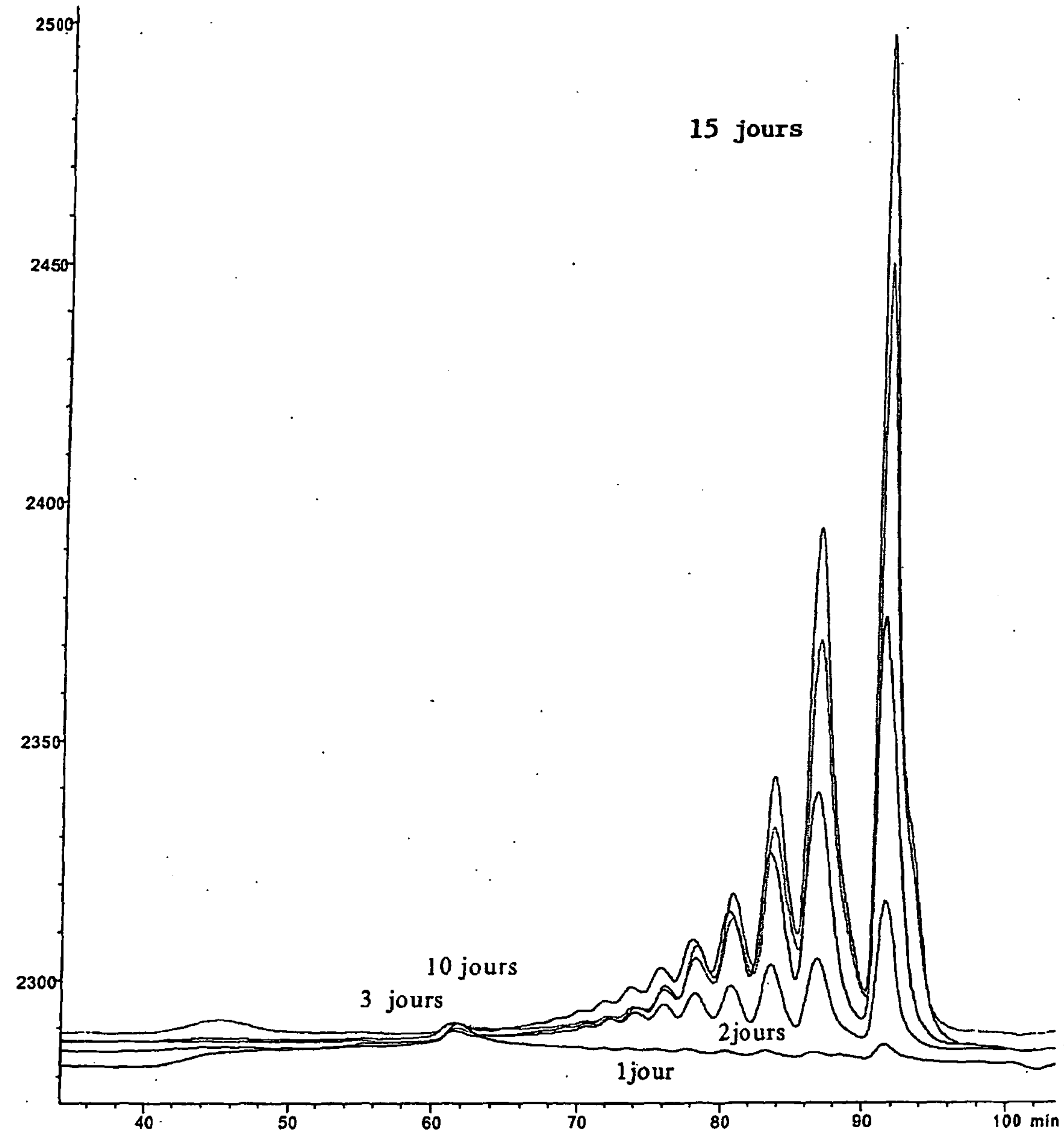


**Figure 1**

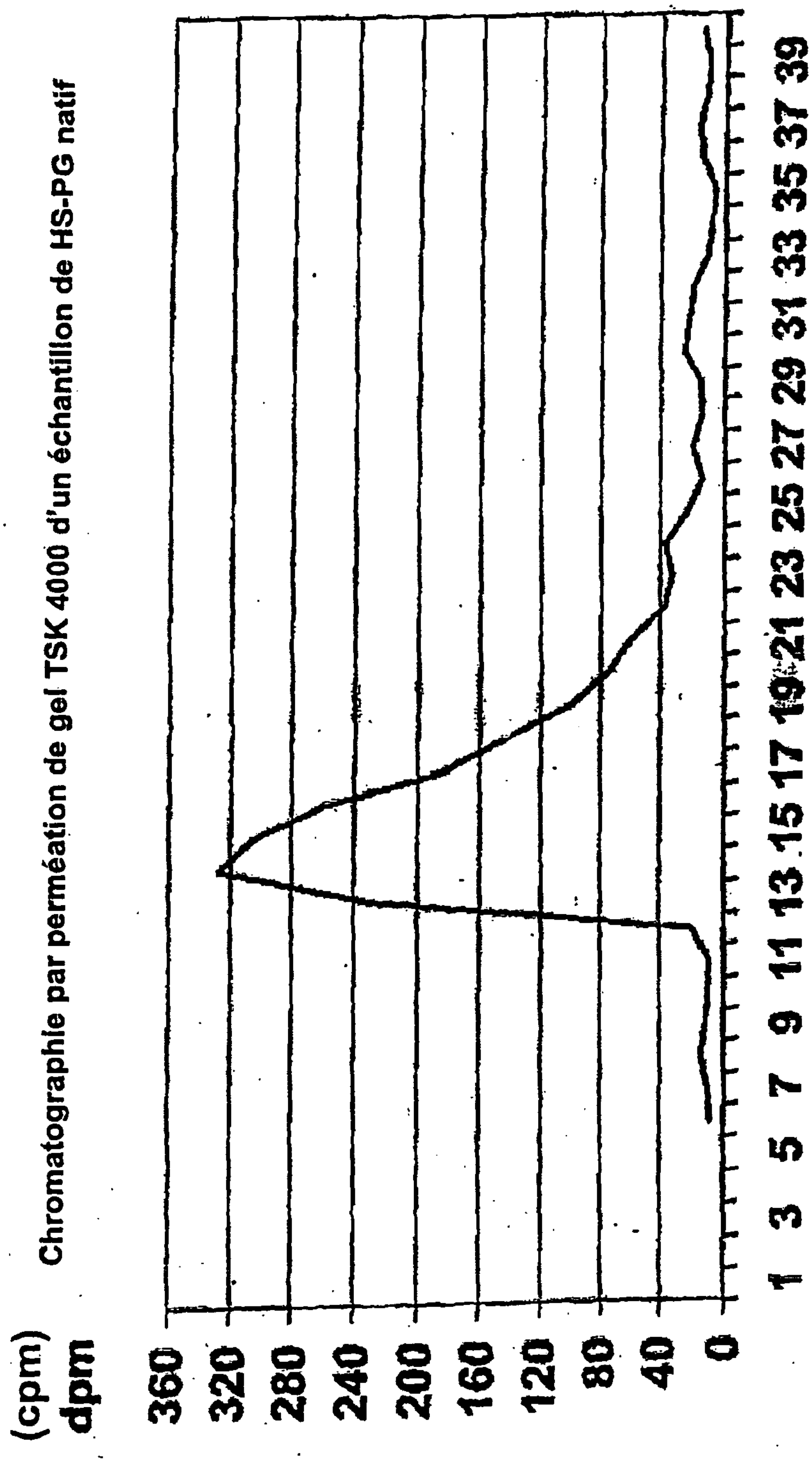
Chromatographie par perméation de gel TSK 4000 d'un échantillon de HS-PG natif
(cpm)
dpm
360
320
280
240
200
160
120
80
40
0
1 3 5 7 9 11 13 15 17 19 21 23 25 27 29 31 33 35 37 39

Figure 1a

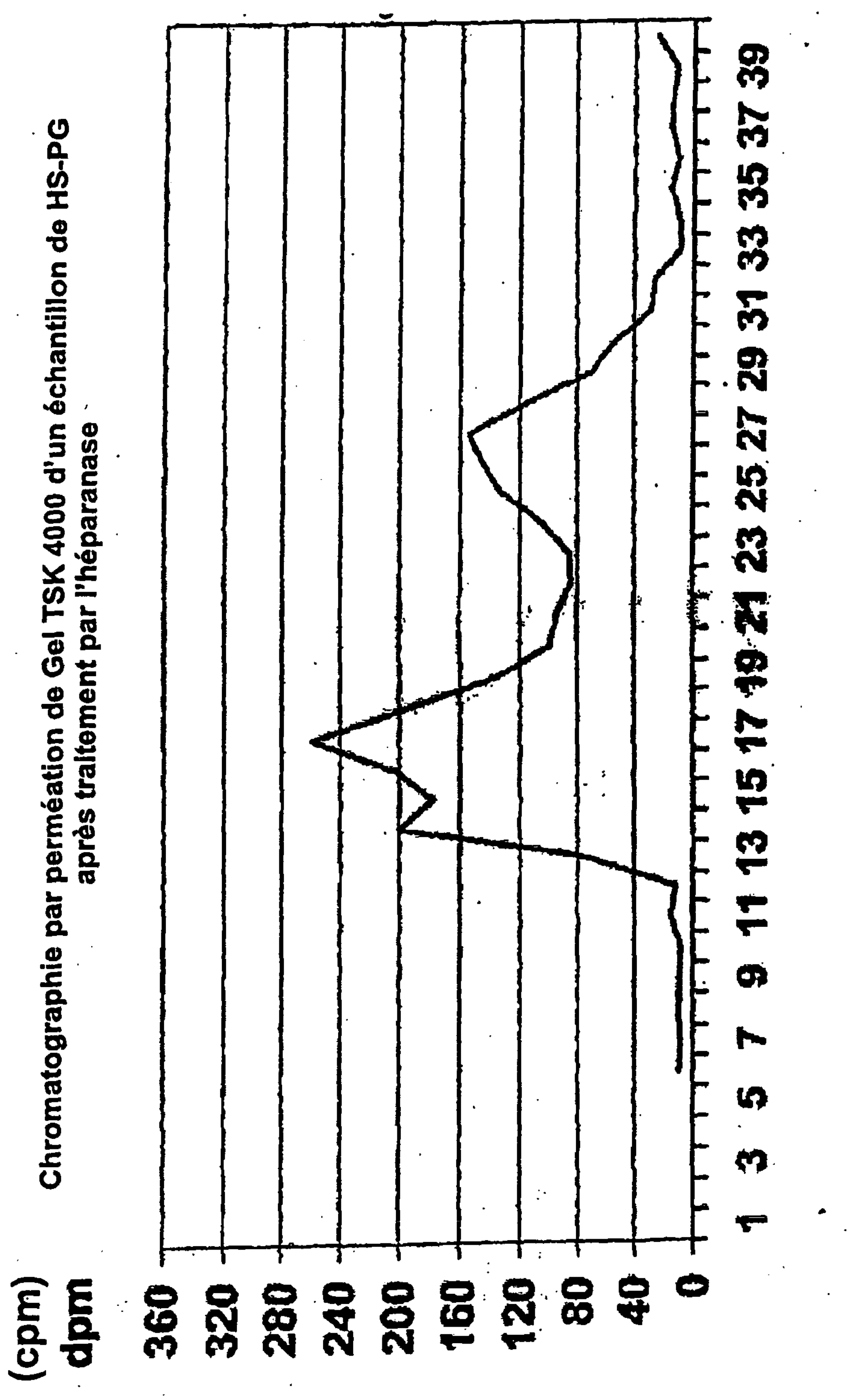
Chromatographie par perméation de Gel TSK 4000 d'un échantillon de HS-PG
après traitement par l'héparanase
(cpm)
dpm
360
320
280
240
200
160
120
80
40
0
1 3 5 7 9 11 13 15 17 19 21 23 25 27 29 31 33 35 37 39

Figure 1b

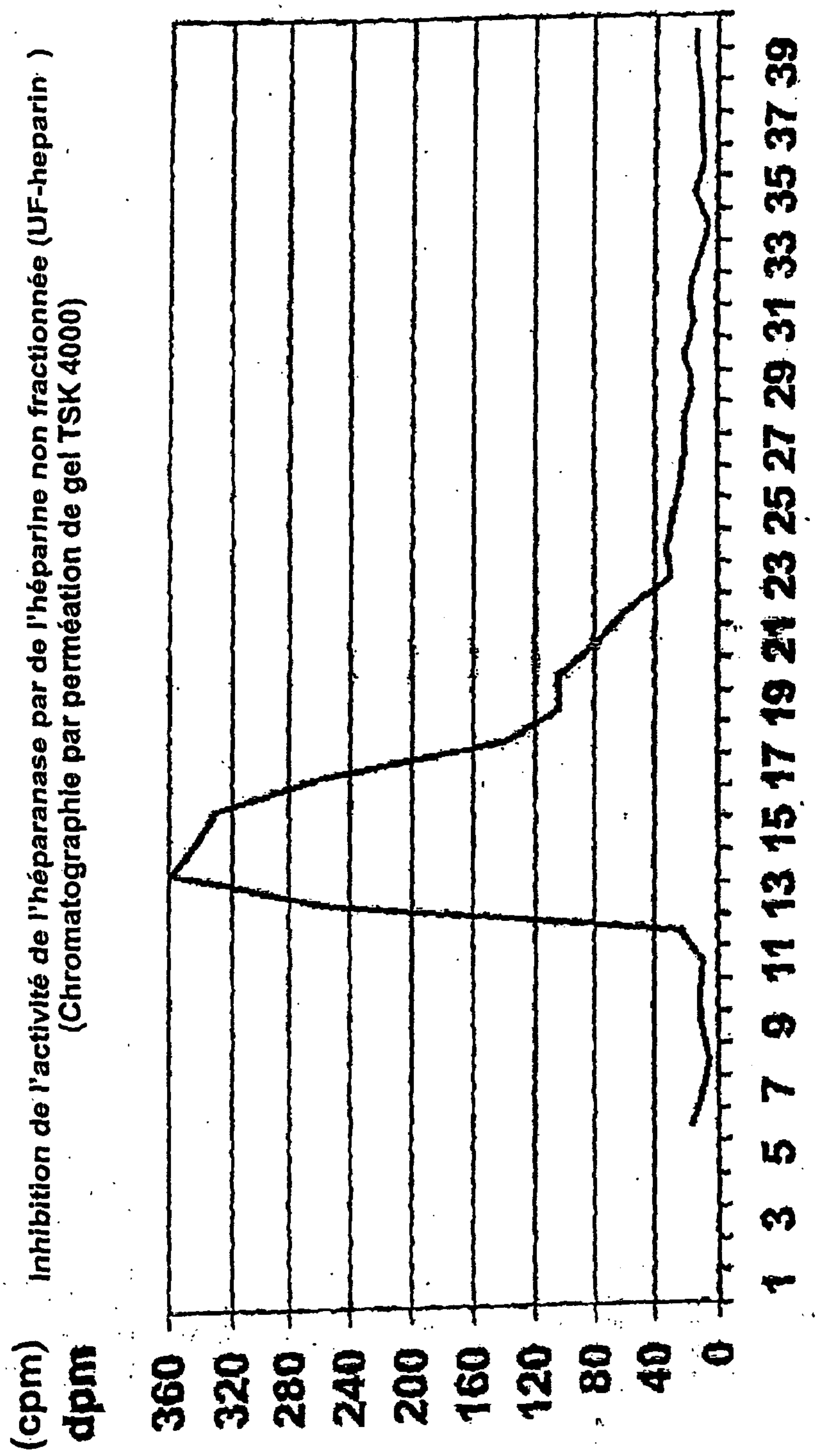
Inhibition de l'activité de l'héparanase par de l'héparine non fractionnée (UF-heparin )
(Chromatographie par perméation de gel TSK 4000)
(cpm)
dpm
360
320
280
240
200
160
120
80
40
0
1 3 5 7 9 11 13 15 17 19 21 23 25 27 29 31 33 35 37 39

Figure 1c

Inhibition de l'activité de l'héparanase par de l'héparine non fractionnée (UF-heparin)

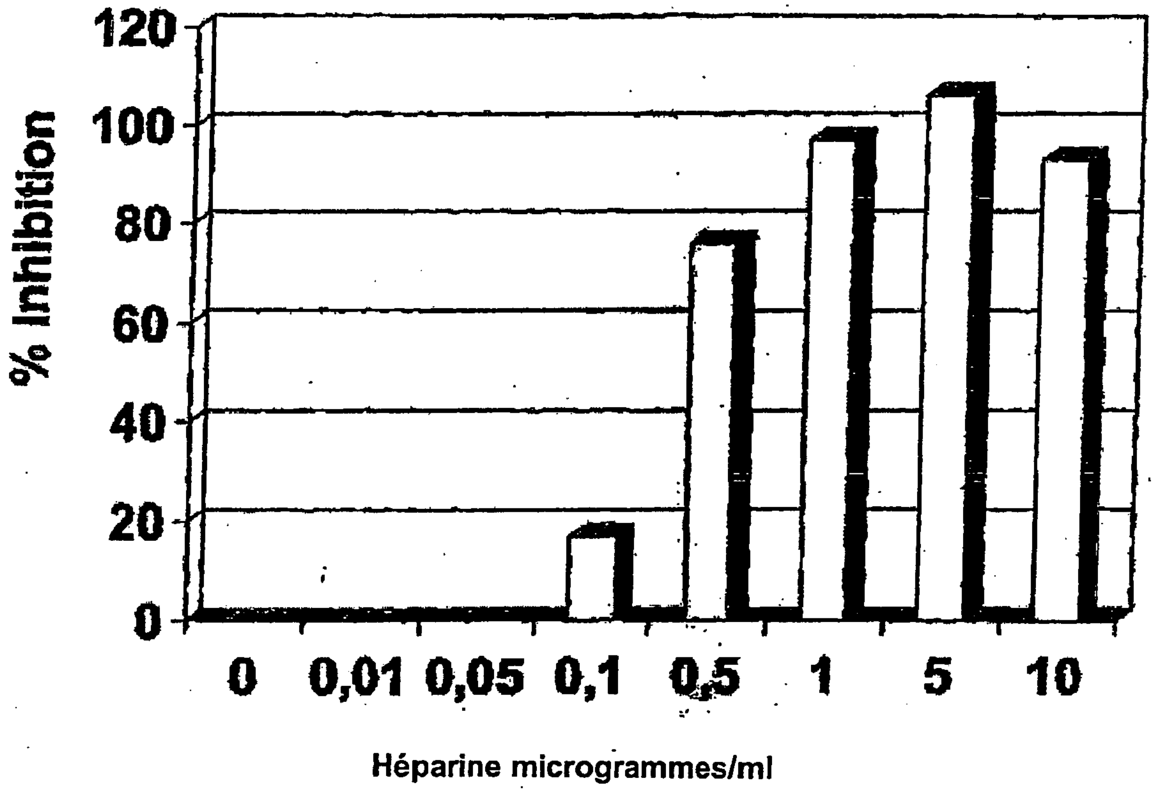


Figure 2

**Suivi de la dépolymérisation de l'héparine par l'héparinase 1 par CTA-SAX**

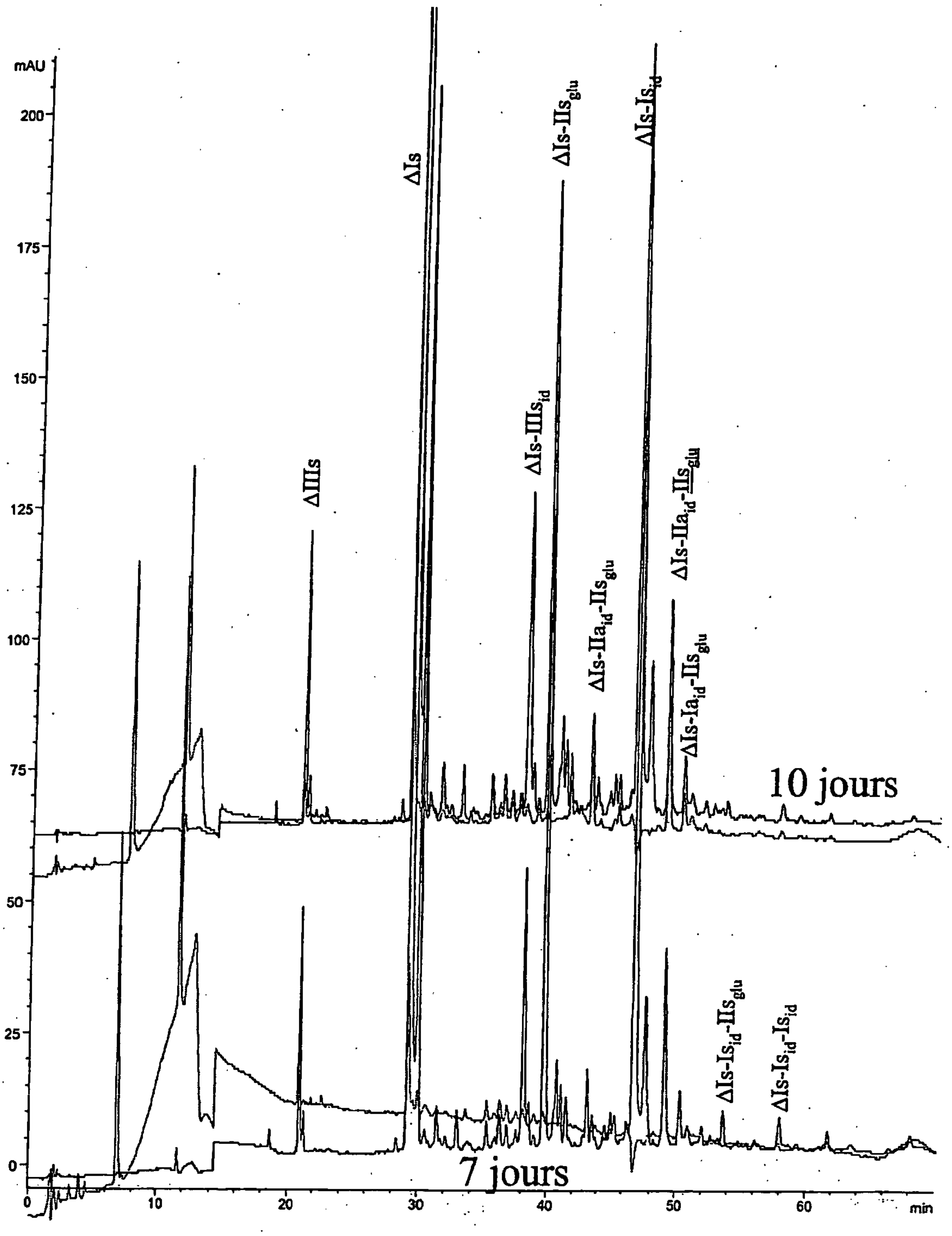


Figure 2a

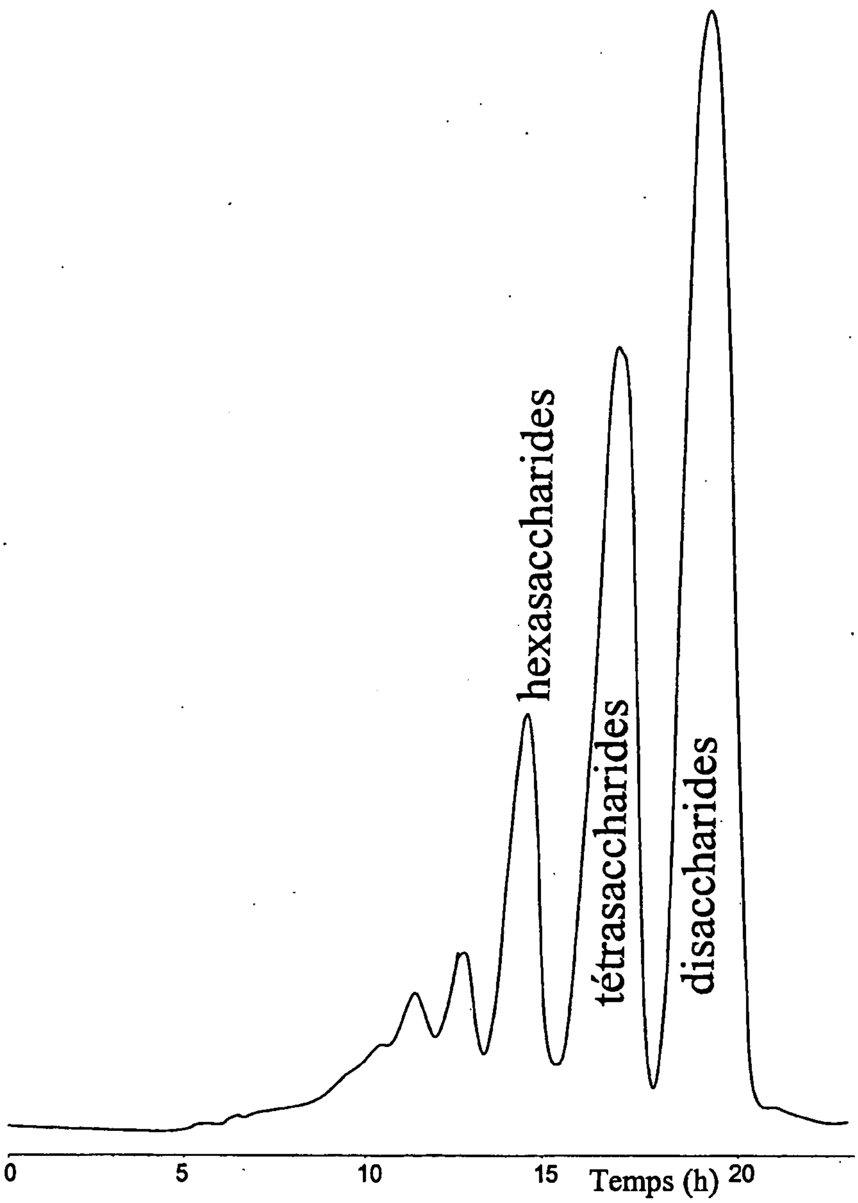


**Fractionnement de 2.5g d'héparine dépolymérisée par GPC (Colonne Biogel P10 (100x5cm) ; Phase mobile : NaCl 0,2N ; débit 80 ml/h ; détection : UV à 250nm. Les signaux à 11 et 12 min. sont respectivement attribués aux fractions décasaccharides et octasaccharides.**

**Figure 3**

Chromatogramme de la fraction hexasaccharide par CTA-SAX

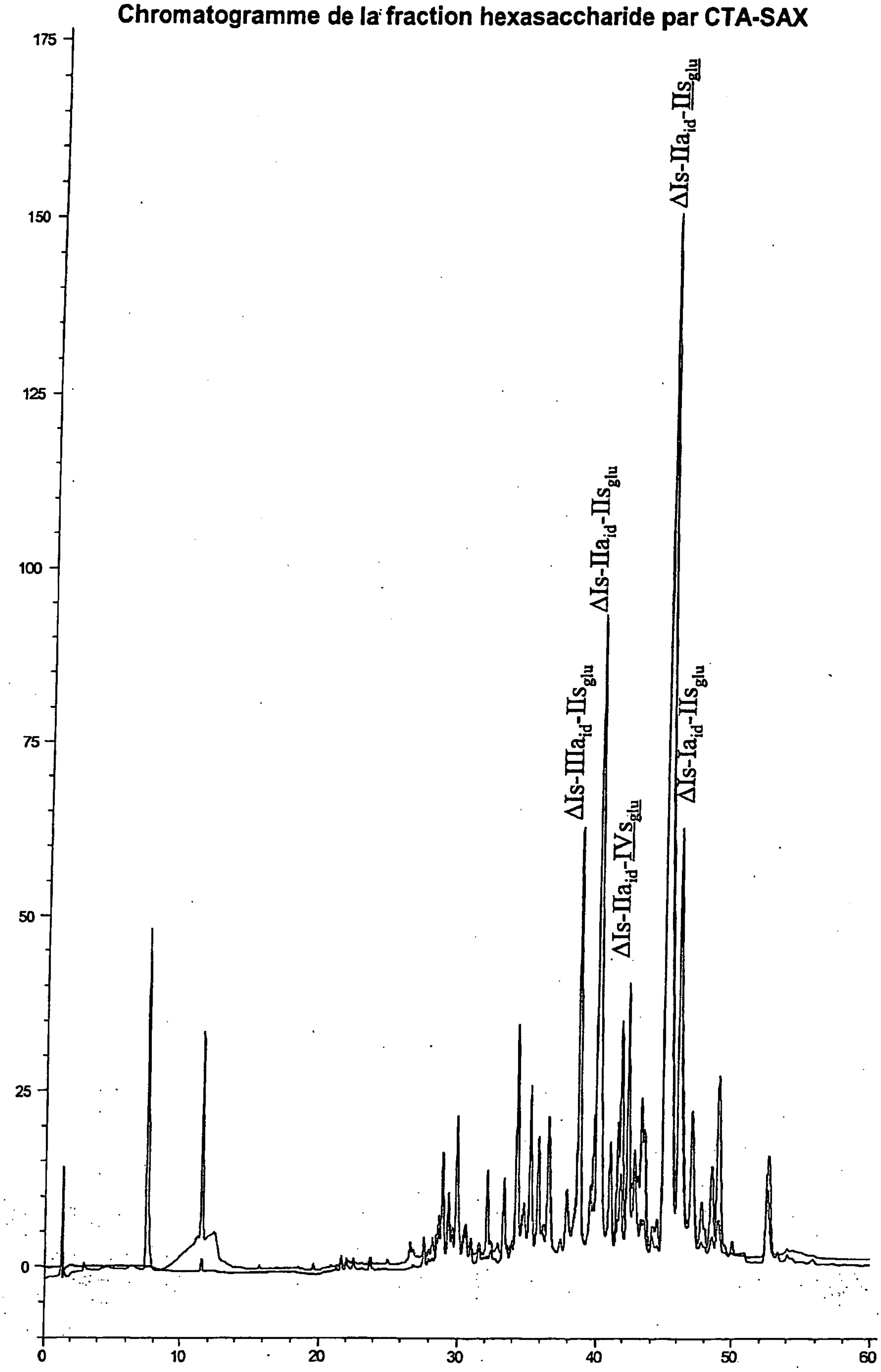


Figure 4

**Séparation de la fraction hexasaccharide par chromatographie semi préparative sur colonne CTA-SAX (25 x 2 cm)**

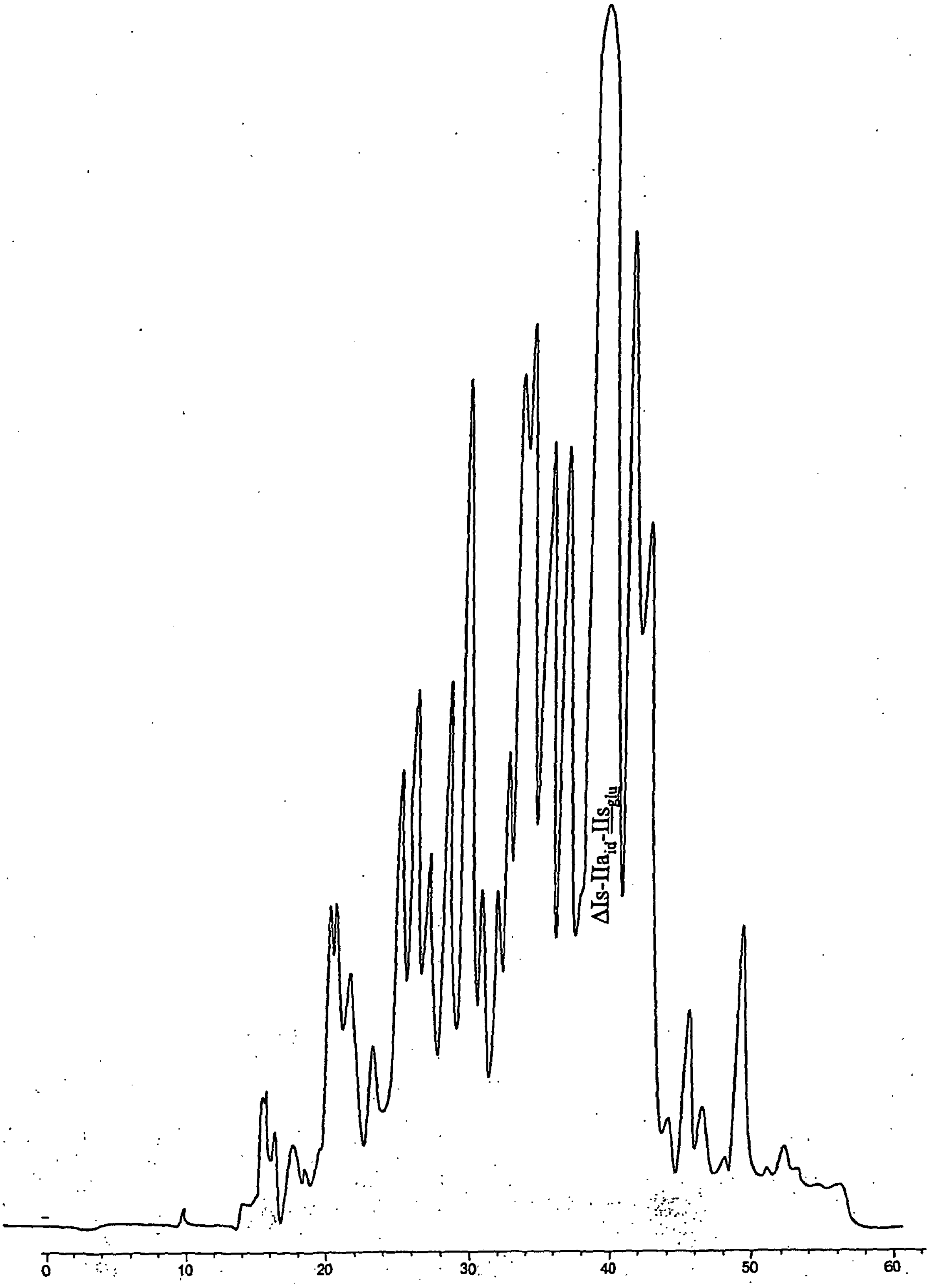


Figure 5

**Chromatogramme final de l'hexasaccharide ΔIs-IIa$_{id}$-IIs$_{glu}$ après dessalage**

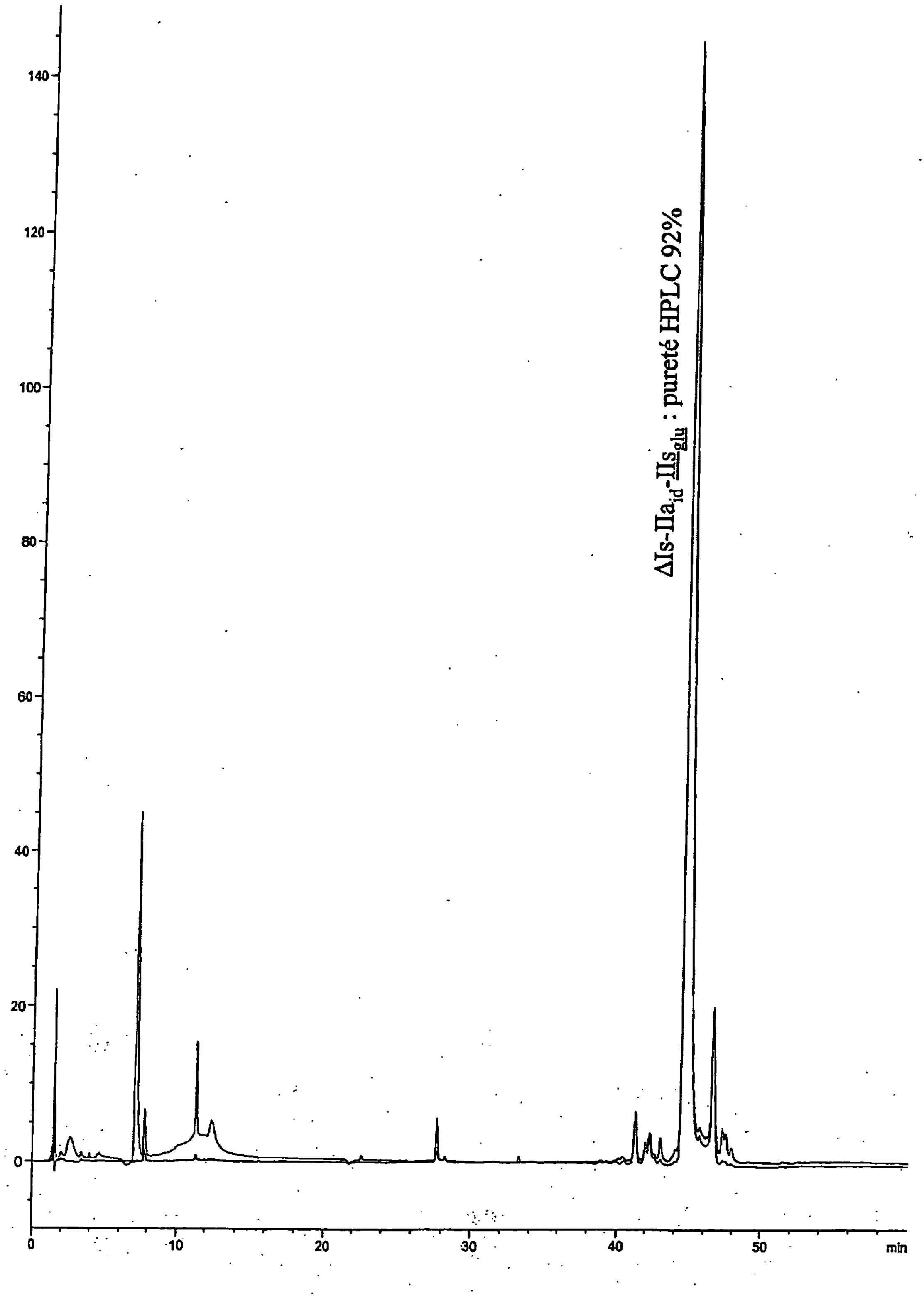


**Figure 6**

Inhibition de l'activité de l'héparanase en fonction de la concentration en Hexa ISO ATIII.

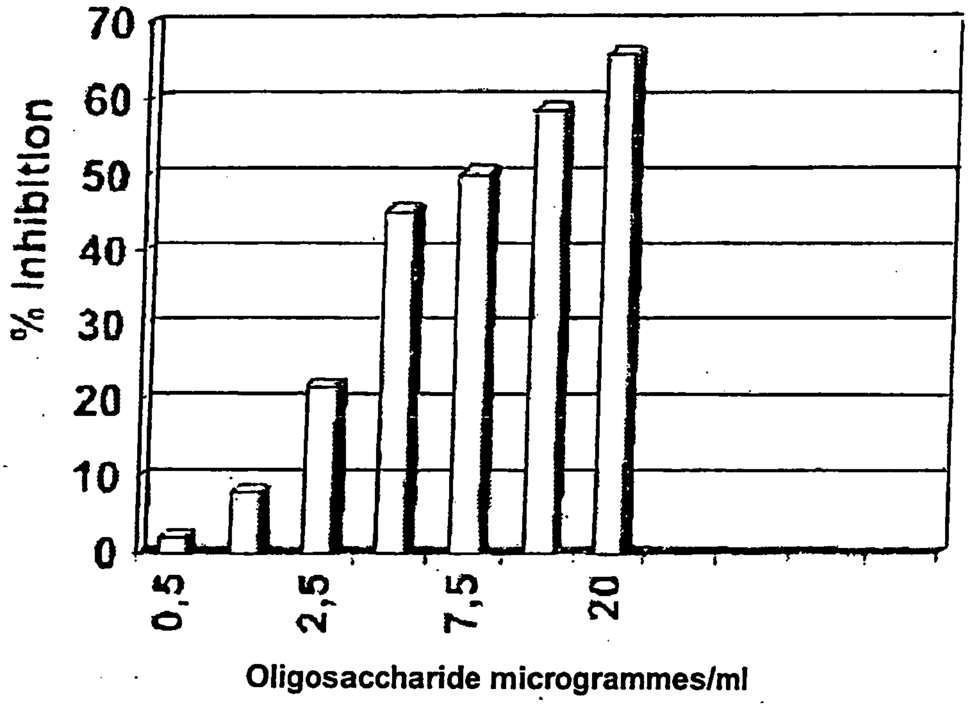


Figure 7

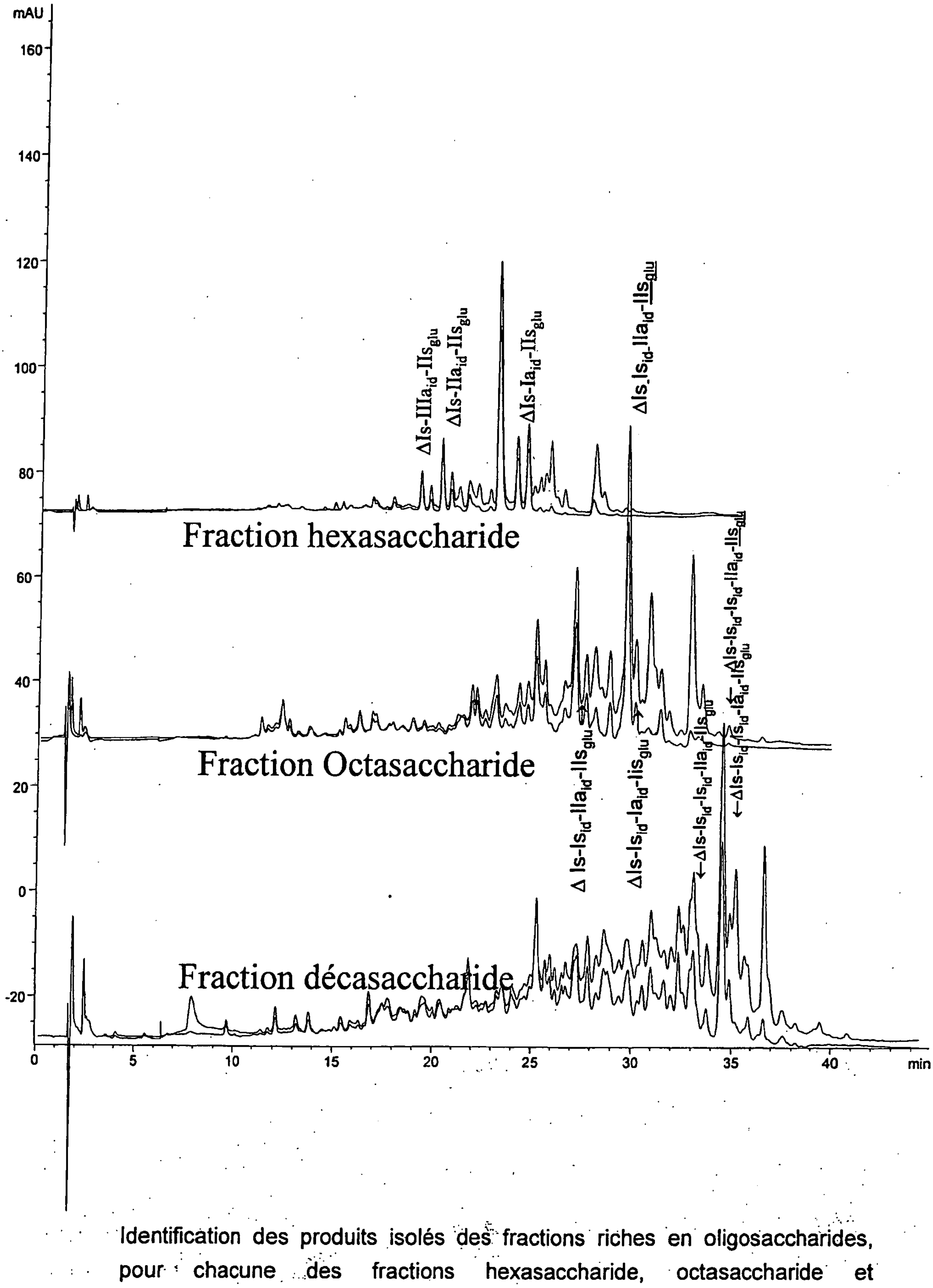


Identification des produits isolés des fractions riches en oligosaccharides, pour chacune des fractions hexasaccharide, octasaccharide et décasaccharide isolées par GPC.

**Figure 8**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- US 4916219 A
- WO 0208295 A
- FR 0302960 W


### Littérature non-brevet citée dans la description


- **BITAN et al.** *Isr. J. Med. Sci.,* 1995, vol. 31, 106-118
- Modulation of neovascularization and metastasis by species of heparin. **VLODAVSKI I et al.** Heparin and related Polysaccharides. Plenum Press, 1992
- **PARISH C R et al.** Evidence that sulphated polysaccharides inhibit tumor metastasis by blocking tumor-cell-derived heparanases. *Int. J. Cancer,* 1987, vol. 40, 511-518
- **PETITOU et al.** *J. Biol. Chem.,* 1988, vol. 263 (18), 8685-8690
- **DRZENIEK et al.** *Blood,* 1999, vol. 93, 2884-2897
- **C.R. PARISH et al.** *Biochim. Biophys. Acta,* 2001, vol. 1471, 99-108
- **M. BARTLETT et al.** *Immunol. Cell Biol.,* 1995, vol. 73, 113-124
- **I. VLODAVSKY et al.** *IMAJ,* 2000, vol. 2, 37-45
- **Y. MATZNER et al.** *J. Clin. Invest.,* 1985, vol. 76, 1306-1313
- **Z. DRZENIEK et al.** *Blood,* 1999, 2884-2897